# EUROPEAN PATENT APPLICATION

(11) **EP 4 803 548 A1**
(43) Date of publication of application: **09.09.2026**
(21) Application number: 24883770.0
(22) Date of filing: 20.03.2024
(51) Int. Cl.: C07K 19/00, C12N 15/62, C12N 15/85, C12N 5/10, G01N 33/68, A61K 39/395, A61K 38/20, A61K 47/68, A61K 39/00, A61P 35/00, A61P 35/02, A61P 31/00, A61P 33/00, A61P 37/02

(54) **ANTIBODIES BINDING TO HLA-A02/HBSAG COMPLEX AND USE THEREOF**

(30) Priority: 02.11.2023 CN 202311448695
(71) Applicant: Beijing Wisdomab Biotechnology Co., Ltd, Beijing 101111 (CN); Genrix(Shanghai) Biopharmaceutical Co., Ltd., Shanghai 201203 (CN); Chongqing Genrix Biopharmaceutical Co., Ltd., Chongqing 401319 (CN)
(72) Inventor: ZHANG, Xueping, Beijing 100176 (CN); LIU, Zhigang, Beijing 100039 (CN); ZHOU, Xiaowei, Beijing 100039 (CN); LIU, Yulan, Beijing 100176 (CN); HAO, Xiaobo, Beijing 100163 (CN); ZHANG, Suhua, Beijing 102600 (CN); HU, Junjie, Beijing 100176 (CN)
(74) Representative: Schnappauf, Georg
(86) International application number: PCT/CN2024/082722
(87) International publication number: WO 2025/091755

(57) **Abstract**

A bispecific antibody, comprising a first antigen-binding fragment that binds to an HLA-A02/HBsAg complex and a second antigen-binding fragment that binds to CD3; in the bispecific antibody, the antibody that binds to the HLA-A02/HBsAg complex is a single domain antibody. A pharmaceutical composition comprising the bispecific antibody or the single domain antibody and use thereof are also disclosed.

## Description

### CROSS REFERECE OF RELATED APPLICATION

The present application claims priority to Chinese Patent Application No. 202311448695.5, filed on November 2, 2023, which is incorporated herein by reference in its entirety.

### TECHNICAL FIELD

The present application pertains to the field of genetic engineering and biomedicine, and more particularly, relates to a bispecific antibody that binds to an HLA-A02/HBsAg complex, a single-domain antibody that binds to the HLA-A02/HBsAg complex, and uses of the bispecific antibody and the single-domain antibody.

### BACKGROUND

Hepatitis B virus (HBV) is the pathogen that causes hepatitis B. Under an electron microscope, the hepatitis B virus can present three forms of particle structures: large spherical particles with a diameter of about 42 nm, small spherical particles with a diameter of about 22 nm, and tubular particles ^{[1, 2]}. The large spherical particles (Dane particles) are complete virions, consisting of an envelope and a nucleocapsid. The envelope contains hepatitis B surface antigen (HBsAg), glycoprotein, and cellular lipids, while the nucleocapsid contains core protein (HBcAg), circular partially double-stranded HBV-DNA, and HBV-DNA polymerase. These particles represent the complete form of the virus and are infectious ^{[3]}.

There are at least 9 genotypes of HBV, from genotype A to I, along with a putative genotype J ^{[4]}. These genotypes show a characteristic geographical distribution: genotype A is primarily prevalent in northern Europe, North America and Central Africa; genotypes B and C are mainly found in Asia; genotype D is predominantly distributed in the Mediterranean region, the Middle East and India; while other genotypes occur in South America and the African continent ^{[5-7]}. In China, among individuals infected with HBV, about 60% carry genotype B, 30% carry genotype C, and a small proportion are infected with genotype D or have mixed genotype B and C infections ^{[8]}.

According to the WHO, in 2019, the global prevalence of HBsAg in the general population was 3.8%, with about 1.5 million new HBV infections, 296 million people living with chronic HBV infection, and 820,000 deaths due to HBV infection-related liver failure, cirrhosis, or hepatocellular carcinoma (HCC). Based on the Polaris International Epidemic Collaboration, the prevalence of HBsAg in the general population in China was 6.1% in 2016, with 86 million individuals chronically infected with HBV ^{[8]}. Currently, there are two main classes of drugs for the treatment of chronic HBV infection in clinical practice: nucleotide analogues (NAs) and interferons ^{[8]}. Depending on different disease manifestations and treatment strategies, a clinical cure rate of about 30% can currently be achieved, but the HBV virus cannot be completely eradicated, and a full cure remains unachievable. Driven by the need for disease control, a number of new drugs, such as antisense RNA, siRNA, capsid assembly inhibitors, monoclonal antibodies targeting HBsAg, therapeutic vaccines, and immune checkpoint inhibitors, are continuously being developed. Monotherapy with most of these agents is not satisfactory, and combination strategies are the primary focus of current clinical research ^{[10]}.

The T cell receptor (TCR), a characteristic surface marker present on all T cells, associates non-covalently with CD3 to form the TCR-CD3 complex. The function of the TCR is to recognize antigens, namely peptide-MHC (p-MHC) complexes. Intracellular proteins are degraded and processed by the proteasome, and the resulting peptides are transported into the endoplasmic reticulum, where they assemble with MHC class I molecules to form complexes. These complexes are then transported through the Golgi apparatus and eventually presented on the cell surface, where they can be recognized by TCRs. Some of these proteins are specifically expressed in tumors or diseased cells and can serve as specific target antigens for therapy. Researchers have found that TCR-mimetic antibodies (TCRm) can be designed to effectively mimic TCRs, recognize MHC complexes presenting tumor-specific antigens, and exert biological activity through T cell-mediated mechanisms ^{[11-13]}. Therefore, the development of TCRm×CD3 bispecific antibodies has become another important field of drug development. Hepatitis B surface antigen (HBsAg) is both a secretory protein and an intracellular protein; its degraded peptides can also be presented on the cell surface ^{[14]}. It has been identified that the HLA-A2-restricted HBsAg₃₄₈₋₃₅₇ (GLSPTVWLSV) peptide can serve as a targeting antigen peptide for drug development ^{[15]}. Given the pressing demand for novel therapeutic strategies and agents, targeting HBsAg-derived antigen peptides may provide a new direction for the complete eradication of hepatitis B virus.

Based on clinical needs, the exploration and development of CD3 bispecific antibodies targeting HBsAg-MHC complexes have important biological and clinical significance.

### SUMMARY OF THE INVENTION

In a first aspect, the present application provides a bispecific antibody comprising a first antigen-binding fragment that binds to an HLA-A02/HBsAg complex and a second antigen-binding fragment that binds to an activated T-cell antigen.

In some embodiments of the first aspect, the bispecific antibody is capable of mediating T cell activation by targeting HLA-A02⁺/HBsAg⁺ tumor cells, and/or the bispecific antibody is capable of mediating the killing of HLA-A02⁺/HBsAg⁺ tumor cells by PBMCs.

In some embodiments of the first aspect, the first antigen-binding fragment comprises HCDR1 as set forth in SEQ ID NO: 32, HCDR2 as set forth in SEQ ID NO: 33, and HCDR3 as set forth in SEQ ID NO: 34; wherein the amino acid sequences of the HCDRs are defined according to the Kabat numbering scheme.

In some embodiments of the first aspect, the first antigen-binding fragment is in the form of a single-domain antibody.

In some embodiments of the first aspect, the first antigen-binding fragment comprises a monovalent or multivalent single-domain antibody that binds to the HLA-A02/HBsAg complex.

In some embodiments of the first aspect, the second antigen-binding fragment comprises HCDR1 as set forth in SEQ ID NO: 37, HCDR2 as set forth in SEQ ID NO: 38, HCDR3 as set forth in SEQ ID NO: 39, LCDR1 as set forth in SEQ ID NO: 40, LCDR2 as set forth in SEQ ID NO: 41, and LCDR3 as set forth in SEQ ID NO: 42; wherein the amino acid sequences of the HCDRs and LCDRs are defined according to the Kabat numbering scheme.

In some embodiments of the first aspect, the second antigen-binding fragment is in the form of a single-chain variable fragment (scFv) or a Fab fragment.

In some embodiments of the first aspect, the first antigen-binding fragment comprises a divalent single-domain antibody that binds to the HLA-A02/HBsAg complex; optionally, the first antigen-binding fragment comprises two heavy chain variable regions from monovalent single-domain antibodies that bind to the HLA-A02/HBsAg complex.

In some embodiments of the first aspect, the first antigen-binding fragment comprises the amino acid sequence as set forth in SEQ ID NOs: 35, 36, 71, or 72; and/or the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 35; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 36; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 71; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 72; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment and the second antigen-binding fragment are linked via an Fc fragment of an antibody heavy chain constant region comprising a first Fc fragment and a second Fc fragment, wherein the first Fc fragment comprises the amino acid C at position 354 and the amino acid W at position 366; or the amino acid C at position 349, the amino acid S at position 366, the amino acid A at position 368, and the amino acid V at position 407; and the second Fc fragment comprises the amino acid C at position 354 and the amino acid W at position 366; or the amino acid C at position 349, the amino acid S at position 366, the amino acid A at position 368, and the amino acid V at position 407; wherein the amino acid positions of the antibody constant region are determined according to the EU numbering scheme.

In some embodiments of the first aspect, the first antigen-binding fragment and the second antigen-binding fragment are linked via an Fc fragment of an antibody heavy chain constant region comprising a first Fc fragment and a second Fc fragment, wherein the first Fc fragment and the second Fc fragment comprise the amino acid F at position 234, the amino acid E at position 235 and the amino acid S at position 331, respectively; wherein the amino acid positions of the antibody constant region are determined according to the EU numbering scheme.

In some embodiments of the first aspect, the first antigen-binding fragment and the second antigen-binding fragment are linked via an Fc fragment of an antibody heavy chain constant region comprising a first Fc fragment and a second Fc fragment, wherein one of the first Fc fragment and the second Fc fragment is linked to the first antigen-binding fragment and the other of the first Fc fragment and the second Fc fragment is linked to the second antigen-binding fragment.

In some embodiments of the first aspect, the bispecific antibody comprises a first arm that binds to the HLA-A02/HBsAg complex and a second arm that binds to the activated T-cell antigen, wherein the first arm comprises the amino acid sequence as set forth in SEQ ID NOs: 45 or 46; and the second arm comprises the amino acid sequence as set forth in SEQ ID NO: 47.

In a second aspect, the present application provides a single-domain antibody that binds to an HLA-A02/HBsAg complex, comprising HCDR1 as set forth in SEQ ID NO: 32, HCDR2 as set forth in SEQ ID NO: 33, and HCDR3 as set forth in SEQ ID NO: 34; wherein the amino acid sequences of the HCDRs are defined according to the Kabat numbering scheme.

In some embodiments of the second aspect, the single-domain antibody binds to the HLA-A02/HBsAg complex at an epitope comprising one or more residues at positions 348-357 of HBsAg as set forth in SEQ ID NO: 51.

In some embodiments of the second aspect, the single-domain antibody is in the form of a monovalent or multivalent single-domain antibody that binds to the HLA-A02/HBsAg complex.

In some embodiments of the second aspect, the single-domain antibody is a bivalent single-domain antibody that binds to the HLA-A02/HBsAg complex.

In some embodiments of the second aspect, the single-domain antibody comprises the amino acid sequence as set forth in SEQ ID NOs: 35, 36, 71, or 72.

In a third aspect, the present application provides a nucleic acid molecule encoding the bispecific antibody of the first aspect or the single-domain antibody of the second aspect.

In a fourth aspect, the present application provides a pharmaceutical composition comprising the bispecific antibody of the first aspect or the single-domain antibody of the second aspect, and a pharmaceutically acceptable excipient, diluent, or carrier.

In a fifth aspect, the present application provides the use of the bispecific antibody of the first aspect, the single-domain antibody of the second aspect, or the pharmaceutical composition of the fourth aspect in the manufacture of a medicament for the prevention or treatment of a disease caused by HBV infection in a patient who is HLA/HBsAg positive.

In a sixth aspect, the present application provides the use of the bispecific antibody of the first aspect or the single-domain antibody of the second aspect in the manufacture of a product for detecting HLA-A02/HBsAg-positive cells.

### BRIEF DESCRIPTION OF THE DRAWINGS

FIG. 1 shows the ELISA results demonstrating that the anti-HLA-A02/HBsAg complex TCRm antibody N1B5 binds to the recombinant protein MIP-H1-01.
FIG. 2 shows the results demonstrating that the anti-HLA-A02/HBsAg complex TCRm antibody N1B5 binds to the HBsAg₃₄₈₋₃₅₇ peptide-pulsed T2 cells.
FIG. 3 shows the results demonstrating that the anti-HLA-A02/HBsAg complex TCRm antibody N1B5 binds to the alanine-scanning mutant peptides of HBsAg₃₄₈₋₃₅₇ (GLSPTVWLSV)-pulsed T2 cells.
FIG. 4 shows the ELISA results demonstrating that the TCRm×CD3 bispecific antibodies recognize both MIP-H1-01 and CD3.
FIG. 5 shows the results demonstrating that TCRm×CD3 bispecific antibodies mediate the activation of Jurkat-Dual cells by targeting the antigen peptide-pulsed T2 cells.
FIG. 6 shows the results demonstrating that the TCRm×CD3 bispecific antibodies mediate the activation of Jurkat-Dual cells by targetingHLA-A02⁺/HBsAg⁺ cells.
FIG. 7 shows the results demonstrating that the TCRm×CD3 bispecific antibodies mediate the killing of the antigen peptide-pulsed T2 cells by PBMCs.
FIG. 8 shows the results demonstrating that the TCRm×CD3 bispecific antibodies mediate the killing of HLA-A02⁺/HBsAg⁺ cells by PBMCs.
FIG. 9 shows the results demonstrating that the TCRm×CD3 bispecific antibody mediates the activation of Jurkat-Dual cells by targeting the alanine-scanning mutant peptides of HBsAg348-357 (GLSPTVWLSV)-pulsed T2 cells.
FIG. 10 shows the results demonstrating that the TCRm×CD3 bispecific antibody mediates the activation of Jurkat-Dual cells by targeting the similar peptides-pulsed T2 cells.
FIG. 11 shows that the TCRm×CD3 bispecific antibody does not mediate the activation of Jurkat-Dual cells by targeting antigen-negative target cells.
FIG. 12 shows the results demonstrating that the TCRm×CD3 bispecific antibody inhibits HepG2-HBsAg tumor cell growth in hPBMC immune reconstruction mice.
FIG. 13 shows a schematic representation of the hydrogen bonds and salt bridges formed in the protein complexes of MIP-H1-01 and N1B5-h4. Amino acid residues involved in these interactions are shown in stick representation.

### DESCRIPTION OF SEQUENCES

SEQ ID NO: 1 shows the amino acid sequence of the HBsAg₃₄₈₋₃₅₇ antigen peptide derived from human (*Homo sapiens*).
SEQ ID NO: 2 shows the amino acid sequence of HBsAg-S1 derived from human (*Homo sapiens*).
SEQ ID NO: 3 shows the amino acid sequence of HBsAg-S2 derived from human (*Homo sapiens*).
SEQ ID NO: 4 shows the amino acid sequence of HBsAg-S3 derived from human (*Homo sapiens*).
SEQ ID NO: 5 shows the amino acid sequence of HBsAg-S4 derived from human (*Homo sapiens*).
SEQ ID NO: 6 shows the amino acid sequence of HBsAg-S5 derived from human (*Homo sapiens*).
SEQ ID NO: 7 shows the amino acid sequence of HBsAg-S6 derived from human (*Homo sapiens*).
SEQ ID NO: 8 shows the amino acid sequence of HBsAg-S7 derived from human (*Homo sapiens*).
SEQ ID NO: 9 shows the amino acid sequence of HBsAg-S8 derived from human (*Homo sapiens*).
SEQ ID NO: 10 shows the amino acid sequence of HBsAg-S9 derived from human (*Homo sapiens*).
SEQ ID NO: 11 shows the amino acid sequence of HBsAg-S10 derived from human (*Homo sapiens*).
SEQ ID NO: 12 shows the amino acid sequence of HBsAg-S11 derived from human (*Homo sapiens*).
SEQ ID NO: 13 shows the amino acid sequence of HBsAg-S12 derived from human (*Homo sapiens*).
SEQ ID NO: 14 shows the amino acid sequence of HBsAg-S13 derived from human (*Homo sapiens*).
SEQ ID NO: 15 shows the amino acid sequence of HBsAg-S14 derived from human (*Homo sapiens*).
SEQ ID NO: 16 shows the amino acid sequence of HBsAg-S15 derived from human (*Homo sapiens*).
SEQ ID NO: 17 shows the amino acid sequence of HBsAg-S16 derived from human (*Homo sapiens*).
SEQ ID NO: 18 shows the amino acid sequence of HBsAg-S17 derived from human (*Homo sapiens*).
SEQ ID NO: 19 shows the amino acid sequence of HBsAg-S18 derived from human (*Homo sapiens*).
SEQ ID NO: 20 shows the amino acid sequence of HBsAg-S19 derived from human (*Homo sapiens*).
SEQ ID NO: 21 shows the amino acid sequence of HBsAg-S20 derived from human (*Homo sapiens*).
SEQ ID NO: 22 shows the amino acid sequence of HBsAg-S21 derived from human (*Homo sapiens*).
SEQ ID NO: 23 shows the amino acid sequence of HBsAg-S22 derived from human (*Homo sapiens*).
SEQ ID NO: 24 shows the amino acid sequence of HBsAg-S23 derived from human (*Homo sapiens*).
SEQ ID NO: 25 shows the amino acid sequence of HBsAg-S24 derived from human (*Homo sapiens*).
SEQ ID NO: 26 shows the amino acid sequence of HBsAg-S25 derived from human (*Homo sapiens*).
SEQ ID NO: 27 shows the amino acid sequence of HBsAg-S26 derived from human (*Homo sapiens*).
SEQ ID NO: 28 shows the amino acid sequence of HBsAg-S27 derived from human (*Homo sapiens*).
SEQ ID NO: 29 shows the amino acid sequence of HBsAg-S28 derived from human (*Homo sapiens*).
SEQ ID NO: 30 shows the amino acid sequence of HBsAg-S29 derived from human (*Homo sapiens*).
SEQ ID NO: 31 shows the amino acid sequence of HBsAg-S30 derived from human (*Homo sapiens*).
SEQ ID NOs: 32-34 show the amino acid sequences of HCDR1, HCDR2, and HCDR3 of the heavy chain variable regions of the camel (*Camelus*) single-domain antibodies N1B5 and N1B5-h4, respectively.
SEQ ID NO: 35 shows the amino acid sequence of the heavy chain variable region of the camel (*Camelus*) single-domain antibody N1B5.
SEQ ID NO: 36 shows the amino acid sequence of the heavy chain variable region of the humanized single-domain antibody N1B5-h4.
SEQ ID NOs: 37-39 show the amino acid sequences of HCDR1, HCDR2, and HCDR3 of the heavy chain variable region of the anti-human CD3 antibody IMCR, respectively.
SEQ ID NOs: 40-42 show the amino acid sequences of LCDR1, LCDR2, and LCDR3 of the light chain variable region of the anti-human CD3 antibody IMCR, respectively.
SEQ ID NO: 43 shows the amino acid sequence of the heavy chain variable region of the anti-human CD3 antibody IMCR.
SEQ ID NO: 44 shows the amino acid sequence of the light chain variable region of the anti-human CD3 antibody IMCR.
SEQ ID NO: 45 shows the amino acid sequence of the arm 2N1B5-G1m3-FcH1n1 in the bispecific antibody 2N1B5+IMCR.
SEQ ID NO: 46 shows the amino acid sequence of the arm 2N1B5-h4-G1m3-FcH1n1 in the bispecific antibody 2N1B5-h4+IMCR.
SEQ ID NO: 47 shows the amino acid sequence of the arm IMCR-anti-CD3-ScFv-G1m3-FcKn1 in 2N1B5-h4+IMCR.
SEQ ID NO: 48 shows the amino acid sequence of human (*Homo sapiens*) CD3E extracellular domain (hCD3E).
SEQ ID NO: 49 shows the amino acid sequence of human (*Homo sapiens*) CD3D extracellular domain (hCD3D).
SEQ ID NO: 50 shows the amino acid sequence of HLA-A02 complex (MIP-H1-01) of GLSPTVWLSV.
SEQ ID NO: 51 shows the amino acid sequence of the hepatitis B surface antigen (HBsAg).
SEQ ID NO: 52 shows the amino acid sequence of the linker.
SEQ ID NO: 53 shows the nucleotide sequence of the primer PCal-CH2R.
SEQ ID NO: 54 shows the amino acid sequence of the His tag.
SEQ ID NO: 55 shows the amino acid sequence of the Fc fragment (mFc) of the mouse (*Mus musculus*) IgG2a antibody.
SEQ ID NO: 56 shows the amino acid sequence of the heavy chain constant region of the human (*Homo sapiens*) IgG1 subtype.
SEQ ID NO: 57 shows the amino acid sequence of IgG1H, a mutant heavy chain constant region of the human IgG1 subtype antibody.
SEQ ID NO: 58 shows the amino acid sequence of IgG1K, a mutant heavy chain constant region of the human IgG1 subtype antibody.
SEQ ID NO: 59 shows the amino acid sequence of IgG1m3-H, a mutant heavy chain constant region of the human IgG1 subtype antibody.
SEQ ID NO: 60 shows the amino acid sequence of IgG1m3-K, a mutant heavy chain constant region of the human IgG1 subtype antibody.
SEQ ID NO: 61 shows the amino acid sequence of IgG1m3-H1n1, a mutant heavy chain constant region of the human IgG1 subtype antibody.
SEQ ID NO: 62 shows the amino acid sequence of IgG1m3-Kn1, a mutant heavy chain constant region of the human IgG1 subtype antibody.
SEQ ID NO: 63 shows the amino acid sequence of the light chain constant region of the human (*Homo sapiens*) λ isotype.
SEQ ID NO: 64 shows the amino acid sequence of the light chain constant region of the human (*Homo sapiens*) κ isotype.
SEQ ID NO: 65 shows the amino acid sequence of the heavy chain variable region (VH) of the germline gene antibody DP47.
SEQ ID NO: 66 shows the amino acid sequence of the light chain variable region (Vκ) of the germline gene antibody DP47.
SEQ ID NO: 67 shows the amino acid sequence of the α chain variable region (Vα) of the control H1-TCR.
SEQ ID NO: 68 shows the amino acid sequence of the β chain variable region (Vβ) of the control H1-TCR.
SEQ ID NO: 69 shows the amino acid sequence of H1-TCRA-Des-G1m3-FcKn1 in the control H1-TCR.
SEQ ID NO: 70 shows the amino acid sequence of H1-TCRB-Des-G1m3-FcH1n1 in the control H1-TCR.
SEQ ID NO: 71 shows the amino acid sequence of the tandemly linked heavy chain variable regions of two camel single-domain antibodies N1B5.
SEQ ID NO: 72 shows the amino acid sequence of the tandemly linked heavy chain variable regions of two camel single-domain antibodies N1B5-h4.
SEQ ID NO: 73 shows the amino acid sequence of IgG1m3-FcH1n1, a mutant Fc fragment of the human IgG1 subtype antibody.
SEQ ID NO: 74 shows the amino acid sequence of the single-chain variable fragment of the anti-human CD3E antibody IMCR.
SEQ ID NO: 75 shows the amino acid sequence of IgG1m3-FcKn1, a mutant Fc fragment of the human IgG1 subtype antibody.
SEQ ID NO: 76 shows the amino acid sequence of IMCR-anti-CD3-scFv-TCRB-Des-G1m3-FcH1n1, the second binding arm of the control bifunctional molecule IMC-I109V.
SEQ ID NO: 77 shows the amino acid sequence of the alanine-scanning mutant peptide at position G1 of HBsAg₃₄₈₋₃₅₇.
SEQ ID NO: 78 shows the amino acid sequence of the alanine-scanning mutant peptide at position L2 of HBsAg₃₄₈₋₃₅₇.
SEQ ID NO: 79 shows the amino acid sequence of the alanine-scanning mutant peptide at position S3 of HBsAg₃₄₈₋₃₅₇.
SEQ ID NO: 80 shows the amino acid sequence of the alanine-scanning mutant peptide at position P4 of HBsAg₃₄₈₋₃₅₇.
SEQ ID NO: 81 shows the amino acid sequence of the alanine-scanning mutant peptide at position T5 of HBsAg₃₄₈₋₃₅₇.
SEQ ID NO: 82 shows the amino acid sequence of the alanine-scanning mutant peptide at position V6 of HBsAg₃₄₈₋₃₅₇.
SEQ ID NO: 83 shows the amino acid sequence of the alanine-scanning mutant peptide at position W7 of HBsAg₃₄₈₋₃₅₇.
SEQ ID NO: 84 shows the amino acid sequence of the alanine-scanning mutant peptide at position L8 of HBsAg₃₄₈₋₃₅₇.
SEQ ID NO: 85 shows the amino acid sequence of the alanine-scanning mutant peptide at position S9 of HBsAg₃₄₈₋₃₅₇.
SEQ ID NO: 86 shows the amino acid sequence of the alanine-scanning mutant peptide at position V10 of HBsAg₃₄₈₋₃₅₇.
SEQ ID NO: 87 shows the amino acid sequence of the irrelevant peptide.
SEQ ID NO: 88 shows the amino acid sequence of N1B5-h4-Twin-Strep-tag.

### DETAILED DESCRIPTION OF THE INVENTION

The inventors of the present application prepared bispecific antibodies (e.g., TCRm bispecific antibodies) by genetically engineering a first antigen-binding fragment that binds to an HLA-A02/HBsAg complex and a second antigen-binding fragment that binds to an activated T-cell antigen (e.g., CD3 molecule). The first antigen-binding fragment binds to the complex of HBsAg₃₄₈₋₃₅₇ (GLSPTVWLSV) and HLA-A02 presented on the surface of a target cell (e.g., HBV-infected cell), and the second antigen-binding fragment binds to the activated T-cell antigen (e.g., CD3 molecule), thereby establishing an interaction between the target cell and the T cell. This interaction causes cytotoxic T-cell activation and lysis of the target cell (e.g., HBV-infected cells) in a major histocompatibility complex (MHC)-dependent manner, achieving the purpose of treating a disease (e.g., a disease caused by HBV infection). In various aspects, the present application provides a novel bispecific antibody comprising a first antigen-binding fragment that binds to an HLA-A02/HBsAg complex and a second antigen-binding fragment that binds to an activated T-cell antigen (e.g., CD3 molecules), a single-domain antibody that binds to an HLA-A02/HBsAg complex, a nucleic acid molecule encoding the bispecific antibody or the single-domain antibody, a vector comprising the nucleic acid molecule, a host cell comprising the nucleic acid molecule or the vector, a method for preparing and purifying the bispecific antibody or the single-domain antibody, and medical and biological applications of the bispecific antibody or the single-domain antibody. According to the sequences of the bispecific antibody or the single-domain antibody provided in the present application, the bispecific antibody or the single-domain antibody that binds to the HLA-A02/HBsAg complex can be constructed as a medicament for clinical use in the prevention or treatment of a disease caused by an HBV infection in which HLA-A02/HBsAg is positive.

Unless otherwise indicated, the inventions of the present application can be implemented using conventional molecular biology, microbiology, cell biology, biochemistry, and immunological techniques known in the art.

Unless otherwise indicated, the terms used in the present application have the meanings commonly understood by those skilled in the art.

### Definitions

The term "HLA-A02/HBsAg complex" as used herein refers to a complex of HLA-A02 molecules with HBsAg. In a specific embodiment of the present application, "HLA-A02/HBsAg complex" refers to an HLA-A02/HBsAg₃₄₈₋₃₅₇ complex formed by an HLA-A02 molecule and the amino acids at positions 348-357 of HBsAg (HBsAg₃₄₈₋₃₅₇) as set forth in SEQ ID NO: 51.

The term "activated T-cell antigen" as used herein refers to an antigen determinant expressed on the surface of T lymphocytes, particularly cytotoxic T lymphocytes, which is capable of inducing T cell activation upon interaction with an antigen-binding molecule. In particular, the interaction between an antigen-binding molecule and an activated T-cell antigen can induce T cell activation by triggering a signaling cascade through T-cell receptor complexes. In a particular aspect, the activated T-cell antigen is the CD3 molecule.

The term "antibody" as used herein refers to an immunoglobulin molecule that is capable of specifically binding to a target via at least one antigen recognition site located in the variable region of the immunoglobulin molecule. Targets include, but are not limited to, carbohydrates, polynucleotides, lipids, polypeptides, and the like. An "antibody" as used herein includes not only an intact *(i.e.,* full-length) antibody, but also a binding fragment thereof (e.g., Fab, Fab', F(ab')₂, or Fv), a variant thereof, a fusion protein comprising antibody portions, a humanized antibody, a chimeric antibody, a bispecific antibody, a linear antibody, a single-chain variable fragment, a single-domain antibody, a multi-specific antibody (e.g., a bispecific antibody), and any other modified configurations of an immunoglobulin molecule comprising a desired specific antigen recognition site, including a glycosylated antibody variant, an amino acid sequence variant of an antibody, and a covalently modified antibody.

Typically, an intact or full-length antibody comprises two heavy chains and two light chains. Each heavy chain contains the heavy chain variable region (VH) and the first, second and third constant regions (CH1, CH2, and CH3). Each light chain contains the light chain variable region (VL) and the constant region (CL). A full-length antibody may belong to any immunoglobulin isotype, such as an IgD, IgE, IgG, IgA, or IgM (or any of the above subclasses) antibody, but it is not necessarily restricted to any particular type. Immunoglobulins can be categorized based on the amino acid sequences of their heavy chain constant regions. Generally, there are five primary isotypes, *i.e.,* IgA, IgD, IgE, IgG, and IgM, some of which can be further classified into subclasses (subtypes), such as IgG1, IgG2, IgG3, IgG4, IgA1, and IgA2. The heavy chain constant regions corresponding to these individual immunoglobulin isotypes are referred to as α, δ, ε, γ, and µ, respectively. Subunit structures and three-dimensional structures of different isotypes of immunoglobulins are well known.

The term "bispecific antibody" as used herein refers to an antibody that has dual binding capacities for two antigen epitopes. The two antigen epitopes may be on different antigens or on the same antigen. Bispecific antibodies may have a variety of structural configurations. For example, a bispecific antibody may consist of two Fc fragments and two binding moieties fused thereto, respectively (similar to a native antibody, except that the two arms bind to different antigen targets or epitopes), and the antigen-binding moieties may be single-domain antibodies, single-chain fragment antibodies (scFvs), or Fab fragments. When two non-overlapping epitopes of the same antigen are targeted, two different binding portions of the bispecific antibody each bind to the N-terminus of one Fc fragment, and the antigen-binding portions of the two arms may be in one of four configurations: single-domain antibody+Fab fragment, single-domain antibody+scFv, scFv+single-domain antibody, and Fab fragment+single-domain antibody. The Fc fragment may contain one or more mutation(s) that ensure heavy chain heteropolymerization, and the KIH technique (knob-in-hole, KIH) is a strategy to address the heavy chain heteropolymerization. Generally, the KIH technique means that by modifying the amino acid sequence of the CH3 region to form a structure that facilitates pairing of heterodimeric hemiantibodies to each other, the structure of a normal antibody can be maintained as much as possible while constituting the bispecific antibody. For guidance on the KIH technique, see, for example, "An efficient route to human bispecific IgG", A. Margaret Merchant et al., Nature Biotechnology, Volume 16, 1998 ^{[16]}, which is incorporated herein by reference in its entirety.

The term "binding portion" or "binding fragment" as used herein is used interchangeably to refer to the portion or region of an intact antibody molecule responsible for binding to an antigen. The antigen-binding domain may comprise a heavy chain variable region (VH), a light chain variable region (VL), or both. Each of the VH and VL regions typically contains three complementarity determining regions (CDR1, CDR2, and CDR3).

It is well known to those skilled in the art that complementarity determining regions (CDRs, usually including CDR1, CDR2 and CDR3) are the regions of a variable region that have the greatest impact on the affinity and specificity of an antibody. The CDR sequences of VH or VL have two common schemes, *i.e.*, the Kabat numbering scheme and the Chothia numbering scheme (see, e.g., Kabat, "Sequences of Proteins of Immunological Interest", National Institutes of Health, Bethesda, Md. (1991) ^{[17]}; A1-Lazikani et al., J. Mol. Biol. 273:927-948 (1997) ^{[18]}; and Martin et al., Proc. Natl. Acad. Sci. USA 86:9268-9272 (1989)) ^{[19]}. For the sequences of the variable regions of a given antibody, the CDR sequences in VH and VL sequences can be determined according to the Kabat numbering scheme or the Chothia numbering scheme. In an embodiment of the present application, the CDR sequences are defined according to the Kabat numbering scheme.

For the sequences of the variable regions of a given antibody, the CDR sequences can be determined in a variety of ways, for example, using the online software Abysis (http://www.abysis.org/).

For typical antibodies, examples of an antigen-binding fragment include, but are not limited to, (1) an Fab fragment, which can be a monovalent fragment consisting of a VL-CL chain and a VH-CH1 chain; (2) an F(ab')₂ fragment, which can be a divalent fragment consisting of two Fab' fragments linked via a disulfide bridge of the hinge region (*i.e.*, a dimer of Fab'); (3) an Fv fragment consisting of VL and VH domains from a single arm of an antibody; (4) a single-chain Fv (scFv), which can be a single polypeptide chain consisting of a VH domain and a VL domain lined by a polypeptide linker; (5) (scFv)₂, which can comprise two VH domains linked via a peptide linker and two VL domains that associate with the two VH domains via disulfide bridges; and (6) single-domain antibody forms.

In the construction of a bispecific antibody, "binding moieties" include, but are not limited to, single-domain antibody forms, Fab fragment forms, and/or single-chain variable fragment (scFv) forms.

The term "single-chain variable fragment (scFv)" as used herein refers to a single-chain antibody generally constructed using genetic engineering techniques, comprising a polypeptide chain that contains a heavy chain variable region (VH) and a light chain variable region (VL). A flexible linker is typically incorporated between the heavy chain variable region and the light chain variable region so that the heavy chain variable region and the light chain variable region can be folded into a correct antigen-binding conformation.

The term "fragment antigen-binding (Fab) fragment", "Fab portion" or a similar term as used herein refers to an antibody fragment capable of binding to an antigen, which is produced by papain digestion of an intact antibody and comprises an intact light chain (VL-CL), a heavy chain variable region, and a CH1 domain (VH-CH1).

The term "single-domain antibody" as used herein refers to the variable domain of a naturally occurring heavy chain antibody that lacks light chains, comprising a heavy chain variable region (VHH) and conventional CH2 and CH3 regions (e.g., one or two groups (heavy chain variable region (VHH) and conventional CH2 and CH3 regions)). The separately cloned and expressed VHH structure maintains structural stability comparable to that of its parent heavy chain antibody and retains antigen-binding activity, representing the smallest known antigen-binding unit. A single-domain antibody is also known as a Nanobody (Nb).

The terms "Fc fragment", "Fc domain", and "Fc portion" as used herein are used interchangeably to refer to the portion of an antibody heavy chain constant region, which includes the hinge region, CH2 domain, and CH3 domain, as defined according to the EU numbering scheme for human IgG1 antibodies.

The term "specific binding" as used herein refers to a non-random binding reaction between two molecules, such as the binding of an antibody to an epitope.

The term "tumor" as used herein refers to a neoplasm or solid lesion formed by abnormal cell growth. The tumor may be benign, premalignant, or malignant. In some embodiments of the present application, the tumor is liver cancer, such as liver cancer caused by HBV infection.

In a first aspect, the present application provides a bispecific antibody comprising a first antigen-binding fragment that binds to an HLA-A02/HBsAg complex and a second antigen-binding fragment that binds to an activated T-cell antigen.

In some embodiments of the first aspect, the bispecific antibody is capable of mediating the activation of T cells by targeting HLA-A02⁺/HBsAg⁺ tumor cells, and/or the bispecific antibody is capable of mediating the killing of HLA-A02⁺/HBsAg⁺ tumor cells by PBMCs.

In some embodiments of the first aspect, the bispecific antibody is capable of mediating the activation of Jurkat-Dual cells by targeting HLA-A02⁺/HBsAg⁺ tumor cells.

In some embodiments of the first aspect, the first antigen-binding fragment binds to the HLA-A02/HBsAg complex at an epitope comprising one or more residues at positions 348-357 of HBsAg as set forth in SEQ ID NO: 51. In some embodiments, the amino acid sequence at positions 348-357 of HBsAg as set forth in SEQ ID NO: 51 is GLSPTVWLSV (SEQ ID NO: 1).

In some embodiments of the first aspect, the first antigen-binding fragment binds to the HLA-A02/HBsAg complex at an epitope comprising at least one of residues at positions 349, 351, 352, 353, 354, 355, 356, and 357 of HBsAg as set forth in SEQ ID NO: 51.

In some embodiments of the first aspect, the first antigen-binding fragment binds to the HLA-A02/HBsAg complex at an epitope comprising residues at positions 349, 352, 353, 354, 355, 356, and 357 of HBsAg as set forth in SEQ ID NO: 51. In some embodiments, the amino acid residues at positions 349, 352, 353, 354, 355, 356, and 357 of HBsAg as set forth in SEQ ID NO: 51 correspond to the amino acid residues at positions 2, 5, 6, 7, 8, 9, and 10 of the HBsAg₃₄₈₋₃₅₇ (GLSPTVWLSV) polypeptide as set forth in SEQ ID NO: 1.

In some embodiments of the first aspect, the first antigen-binding fragment binds to the HLA-A02/HBsAg complex at an epitope comprising residues at positions 351, 352, 353, 354, 355, 356, and 357 of HBsAg as set forth in SEQ ID NO: 51. In some embodiments, the amino acid residues at positions 351, 352, 353, 354, 355, 356, and 357 of HBsAg as set forth in SEQ ID NO: 51 correspond to the amino acid residues at positions 4, 5, 6, 7, 8, 9, and 10 of the HBsAg₃₄₈₋₃₅₇ (GLSPTVWLSV) polypeptide as set forth in SEQ ID NO: 1.

In some embodiments of the first aspect, the first antigen-binding fragment binds to the HLA-A02/HBsAg complex at an epitope comprising residues at positions 349, 351, 352, 353, 354, 355, 356, and 357 of HBsAg as set forth in SEQ ID NO: 51. In some embodiments, the amino acid residues at positions 349, 351, 352, 353, 354, 355, 356, and 357 of HBsAg as set forth in SEQ ID NO: 51 correspond to amino acid residues at positions 2, 4, 5, 6, 7, 8, 9, and 10 of the HBsAg₃₄₈₋₃₅₇ (GLSPTVWLSV) polypeptide as set forth in SEQ ID NO: 1.

In some embodiments of the first aspect, the activated T-cell antigen is the CD3 molecule.

In some embodiments of the first aspect, the first antigen-binding fragment comprises HCDR1 as set forth in SEQ ID NO: 32, HCDR2 as set forth in SEQ ID NO: 33, and HCDR3 as set forth in SEQ ID NO: 34; wherein the amino acid sequences of the HCDRs are defined according to the Kabat numbering scheme.

In some embodiments of the first aspect, the first antigen-binding fragment is in the form of a single-domain antibody.

In some embodiments of the first aspect, the first antigen-binding fragment comprises a monovalent or multivalent (e.g., 1, 2, 3, 4, 5, or 6-valent) single-domain antibody that binds to the HLA-A02/HBsAg complex.

In some embodiments of the first aspect, the first antigen-binding fragment comprises a divalent single-domain antibody that binds to the HLA-A02/HBsAg complex. In some embodiments, the first antigen-binding fragment comprises two heavy chain variable regions from monovalent single-domain antibodies that bind to the HLA-A02/HBsAg complex. In some embodiments, the first antigen-binding fragment comprises two heavy chain variable regions from monovalent single-domain antibodies that bind to the HLA-A02/HBsAg complex, which are linked via direct fusion. In some embodiments, the first antigen-binding fragment comprises two heavy chain variable regions from monovalent single-domain antibodies that bind to the HLA-A02/HBsAg complex, which are linked via a linker. In some embodiments, the linker is a linker comprising a GS-type flexible peptide, such as (GGGGS)ₙ, where n is an integer ≥ 1. In some specific embodiments, the linker is GGGGSGGGGSGGGGS (SEQ ID NO: 52).

In some embodiments of the first aspect, the first antigen-binding fragment comprises the amino acid sequence as set forth in SEQ ID NO: 35.

In some embodiments of the first aspect, the first antigen-binding fragment comprises the amino acid sequence as set forth in SEQ ID NO: 36.

In some embodiments of the first aspect, the first antigen-binding fragment comprises the amino acid sequence as set forth in SEQ ID NO: 71.

In some embodiments of the first aspect, the first antigen-binding fragment comprises the amino acid sequence as set forth in SEQ ID NO: 72.

In some embodiments of the first aspect, the amino acid sequence as set forth in SEQ ID NO: 71 comprises two amino acid sequences as set forth in SEQ ID NO: 35.

In some embodiments of the first aspect, the amino acid sequence as set forth in SEQ ID NO: 72 comprises two amino acid sequences as set forth in SEQ ID NO: 36.

In some embodiments of the first aspect, the second antigen-binding fragment comprises HCDR1 as set forth in SEQ ID NO: 37, HCDR2 as set forth in SEQ ID NO: 38, HCDR3 as set forth in SEQ ID NO: 39, LCDR1 as set forth in SEQ ID NO: 40, LCDR2 as set forth in SEQ ID NO: 41, and LCDR3 as set forth in SEQ ID NO: 42; wherein the amino acid sequences of the HCDRs are defined according to the Kabat numbering scheme.

In some embodiments of the first aspect, the second antigen-binding fragment is in the form of a single-chain variable fragment (scFv) or a Fab fragment.

In some embodiments of the first aspect, the second antigen-binding fragment is in the form of an scFv.

In some embodiments of the first aspect, the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 35; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 36; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 71; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises the amino acid sequence as set forth in SEQ ID NO: 72; and the second antigen-binding fragment comprises a heavy chain variable region having the amino acid sequence as set forth in SEQ ID NO: 43 and a light chain variable region having the amino acid sequence as set forth in SEQ ID NO: 44.

In some embodiments of the first aspect, the first antigen-binding fragment comprises an amino acid sequence that differs from the amino acid sequence as set forth in SEQ ID NOs: 35, 36, 71, or 72 by about 1, 2, 3, 4, 5, 6, 7, 8, 9, or 10 amino acid substitutions, deletions, and/or additions.

In some embodiments of the first aspect, the first antigen-binding fragment comprises an amino acid sequence that has at least 90%, 91%, 92%, 93%, 94%, 95%, 96%, 97%, 98%, 99%, or higher identity to the amino acid sequence as set forth in SEQ ID NOs: 35, 36, 71, or 72.

In some embodiments of the first aspect, the amino acid sequence as set forth in SEQ ID NOs: 35, 36, 71, or 72 may also be truncated at the C-terminus or N-terminus by about 1, 2, 3, 4, 5, 6, 7, 8, 9, 10, 11, 12, 13, 14, 15, 20, 25, or more amino acids, while still retaining the function similar to that of the first antigen-binding fragment.

In some embodiments of the first aspect, the amino acid sequence as set forth in SEQ ID NOs: 35, 36, 71, or 72 may also have 1, 2, 3, 4, 5, 6, 7, 8, 9, 10, 11, 12, 13, 14, 15, 20, 25, or more amino acids added at the C-terminus or N-terminus, while still retaining the function similar to that of the first antigen-binding fragment.

In some embodiments of the first aspect, the amino acid sequence as set forth in SEQ ID NOs: 35, 36, 71, or 72 may also have 1, 2, 3, 4, 5, 6, 7, 8, 9, 10, 11, 12, 13, 14, 15, 20, 25, or more amino acids added or deleted at regions other than the C-terminus or the N-terminus, provided that the altered amino acid sequence substantially retains the function similar to that of the first antigen-binding fragment.

In some embodiments of the first aspect, the second antigen-binding fragment comprises a heavy chain variable region having the amino acid sequence as set forth in SEQ ID NO: 43.

In some embodiments of the first aspect, the second antigen-binding fragment comprises a heavy chain variable region having an amino acid sequence that differs from the amino acid sequence as set forth in SEQ ID NO: 43 by about 1, 2, 3, 4, 5, 6, 7, 8, 9, or 10 amino acid substitutions, deletions, and/or additions.

In some embodiments of the first aspect, the second antigen-binding fragment comprises a heavy chain variable region having an amino acid sequence that has at least 90%, 91%, 92%, 93%, 94%, 95%, 96%, 97%, 98%, 99%, or higher identity to the amino acid sequence as set forth in SEQ ID NO: 43.

In some embodiments of the first aspect, the amino acid sequence as set forth in SEQ ID NO: 43 may also be truncated at the C-terminus or N-terminus by about 1, 2, 3, 4, 5, 6, 7, 8, 9, 10, 11, 12, 13, 14, 15, 20, 25, or more amino acids, while still retaining the function similar to that of the heavy chain variable region of the second antigen-binding fragment.

In some embodiments of the first aspect, the amino acid sequence as set forth in SEQ ID NO: 43 may also have 1, 2, 3, 4, 5, 6, 7, 8, 9, 10, 11, 12, 13, 14, 15, 20, 25, or more amino acids added at the C-terminus or N-terminus, while still retaining the function similar to that of the heavy chain variable region of the second antigen-binding fragment.

In some embodiments of the first aspect, the amino acid sequence as set forth in SEQ ID NO: 43 may also have 1, 2, 3, 4, 5, 6, 7, 8, 9, 10, 11, 12, 13, 14, 15, 20, 25, or more amino acids added or deleted at regions other than the C-terminus or the N-terminus, provided that the altered amino acid sequence substantially retains the function similar to that of the heavy chain variable region of the second antigen-binding fragment.

In some embodiments of the first aspect, the second antigen-binding fragment comprise a light chain variable region having the amino acid sequence as set forth in SEQ ID NO: 44.

In some embodiments of the first aspect, the second antigen-binding fragment comprises a light chain variable region having an amino acid sequence that differs from the amino acid sequence as set forth in SEQ ID NO: 44 by about 1, 2, 3, 4, 5, 6, 7, 8, 9, or 10 amino acid substitutions, deletions, and/or additions.

In some embodiments of the first aspect, the second antigen-binding fragment comprise a light chain variable region having an amino acid sequence that has at least 90%, 91%, 92%, 93%, 94%, 95%, 96%, 97%, 98%, 99%, or higher identity to the amino acid sequence as set forth in SEQ ID NO: 44.

In some embodiments of the first aspect, the amino acid sequence as set forth in SEQ ID NO: 44 may also be truncated at the C-terminus or N-terminus by about 1, 2, 3, 4, 5, 6, 7, 8, 9, 10, 11, 12, 13, 14, 15, 20, 25, or more amino acids, while still retaining the function similar to that of the light chain variable region of the second antigen-binding fragment.

In some embodiments of the first aspect, the amino acid sequence as set forth in SEQ ID NO: 44 may also have 1, 2, 3, 4, 5, 6, 7, 8, 9, 10, 11, 12, 13, 14, 15, 20, 25, or more amino acids added at the C-terminus or N-terminus, while still retaining the function similar to that of the light chain variable region of the second antigen-binding fragment.

In some embodiments of the first aspect, the amino acid sequence as set forth in SEQ ID NO: 44 may also have 1, 2, 3, 4, 5, 6, 7, 8, 9, 10, 11, 12, 13, 14, 15, 20, 25, or more amino acids may also be added or deleted at regions other than the C-terminus or the N-terminus, provided that the altered amino acid sequence substantially retains the function similar to that of the light chain variable region of the second antigen-binding fragment.

In some embodiments of the first aspect, the first antigen-binding fragment and the second antigen-binding fragment are linked via an Fc fragment of an antibody heavy chain constant region comprising a first Fc fragment and a second Fc fragment, wherein amino acids at positions 354 and 366 of the first Fc fragment are C and W, respectively, or amino acids at positions 349, 366, 368, and 407 of the first Fc fragment are C, S, A, and V, respectively; and amino acids at positions 354 and 366 of the second Fc fragment are C and W, respectively, or amino acids at positions 349, 366, 368, and 407 of the second Fc fragment are C, S, A, and V, respectively; wherein the amino acid positions of the antibody constant regions are determined according to the EU numbering scheme.

In some embodiments of the first aspect, the first antigen-binding fragment and the second antigen-binding fragment are linked via an Fc fragment of an antibody heavy chain constant region comprising a first Fc fragment and a second Fc fragment, wherein amino acids at positions 354 and 366 of the first Fc fragment are C and W, respectively, and amino acids at positions 349, 366, 368, and 407 of the second Fc fragment are C, S, A, and V, respectively; wherein the amino acid positions of the antibody constant regions are determined according to the EU numbering scheme.

In some embodiments of the first aspect, the first antigen-binding fragment and the second antigen-binding fragment are linked via an Fc fragment of an antibody heavy chain constant region comprising a first Fc fragment and a second Fc fragment, wherein amino acids at positions 234, 235, and 331 of the first Fc fragment and the second Fc fragment are F, E, and S, respectively; wherein the amino acid positions of the antibody constant regions are determined according to the EU numbering scheme.

In some embodiments of the first aspect, the first antigen-binding fragment and the second antigen-binding fragment are linked via an Fc fragment of an antibody heavy chain constant region comprising a first Fc fragment and a second Fc fragment, wherein one of the first Fc fragment and the second Fc fragment is linked to the first antigen-binding fragment and the other of the first Fc fragment and the second Fc fragment is linked to the second antigen-binding fragment.

In some embodiments of the first aspect, the first antigen-binding fragment (e.g., at the C-terminus) is linked to the N-terminus of the first Fc fragment (e.g., the amino acid sequence as set forth in SEQ ID NO: 73).

In some embodiments of the first aspect, the second antigen-binding fragment (e.g., at the C-terminus) is linked to the N-terminus of the second Fc fragment (e.g., the amino acid sequence as set forth in SEQ ID NO: 75).

In some embodiments of the first aspect, the Fc fragment of the antibody heavy chain constant region is an Fc fragment of the IgG1 subtype. In some embodiments, the first Fc fragment is an Fc fragment of the IgG1 subtype; and/or the second Fc fragment is an Fc fragment of the IgG1 subtype.

In some embodiments of the first aspect, the Fc fragment of the antibody heavy chain constant region is an Fc fragment of the IgG1m3 allotype. In some embodiments, the first Fc fragment is an Fc fragment of the IgG1m3 allotype; and/or the second Fc fragment is an Fc fragment of the IgG1m3 allotype.

In some embodiments of the first aspect, the bispecific antibody comprises a first arm that binds to the HLA-A02/HBsAg complex and a second arm that binds to the activated T-cell antigen, wherein the first arm comprises the amino acid sequence as set forth in SEQ ID NOs: 45 or 46; and the second arm comprises the amino acid sequence as set forth in SEQ ID NO: 47.

In some embodiments of the first aspect, the first arm comprises the amino acid sequence as set forth in SEQ ID NOs: 45 or 46.

In some embodiments of the first aspect, the first arm comprises an amino acid sequence that differs from the amino acid sequence as set forth in SEQ ID NOs: 45 or 46 by about 1, 2, 3, 4, 5, 6, 7, 8, 9, or 10 amino acid substitutions, deletions, and/or additions.

In some embodiments of the first aspect, the first arm comprises an amino acid sequence that has at least 90%, 91%, 92%, 93%, 94%, 95%, 96%, 97%, 98%, 99%, or higher identity to the amino acid sequence as set forth in SEQ ID NOs: 45 or 46.

In some embodiments of the first aspect, the amino acid sequence as set forth in SEQ ID NOs: 45 or 46 may also be truncated at the C-terminus or N-terminus by about 1, 2, 3, 4, 5, 6, 7, 8, 9, 10, 11, 12, 13, 14, 15, 20, 25, or more amino acids, while still retaining the function similar to that of the first arm.

In some embodiments of the first aspect, the amino acid sequence as set forth in SEQ ID NOs: 45 or 46 may also have 1, 2, 3, 4, 5, 6, 7, 8, 9, 10, 11, 12, 13, 14, 15, 20, 25, or more amino acids added at the C-terminus or N-terminus, while still retaining the function similar to that of the first arm.

In some embodiments of the first aspect, the amino acid sequence as set forth in SEQ ID NOs: 45 or 46 may also have 1, 2, 3, 4, 5, 6, 7, 8, 9, 10, 11, 12, 13, 14, 15, 20, 25, or more amino acids added or deleted at regions other than the C-terminus or the N-terminus, provided that the altered amino acid sequence substantially retains the function similar to that of the first arm.

In some embodiments of the first aspect, the second arm comprises the amino acid sequence as set forth in SEQ ID NO: 47.

In some embodiments of the first aspect, the second arm comprises an amino acid sequence that differs from the amino acid sequence as set forth in SEQ ID NO: 47 by about 1, 2, 3, 4, 5, 6, 7, 8, 9, or 10 amino acid substitutions, deletions, and/or additions.

In some embodiments of the first aspect, the second arm comprises an amino acid sequence that has at least 90%, 91%, 92%, 93%, 94%, 95%, 96%, 97%, 98%, 99%, or higher identity to the amino acid sequence as set forth in SEQ ID NO: 47.

In some embodiments of the first aspect, the amino acid sequence as set forth in SEQ ID NO: 47 may also be truncated by about 1, 2, 3, 4, 5, 6, 7, 8, 9, 10, 11, 12, 13, 14, 15, 20, 25, or more amino acids at the C-terminus or N-terminus, while still retaining the function similar to that of the second arm.

In some embodiments of the first aspect, the amino acid sequence as set forth in SEQ ID NO: 47 may also have 1, 2, 3, 4, 5, 6, 7, 8, 9, 10, 11, 12, 13, 14, 15, 20, 25, or more amino acids added at the C-terminus or N-terminus, while still retaining the function similar to that of the second arm.

In some embodiments of the first aspect, the amino acid sequence as set forth in SEQ ID NO: 47 may have 1, 2, 3, 4, 5, 6, 7, 8, 9, 10, 11, 12, 13, 14, 15, 20, 25, or more amino acidsadded or deleted at regions other than the C-terminus or the N-terminus, provided that the altered amino acid sequence substantially retains the function similar to that of the second arm.

In a second aspect, the present application provides a single-domain antibody that binds to an HLA-A02/HBsAg complex, comprising HCDR1 as set forth in SEQ ID NO: 32, HCDR2 as set forth in SEQ ID NO: 33, and HCDR3 as set forth in SEQ ID NO: 34; wherein the amino acid sequences of the HCDRs are defined according to the Kabat numbering scheme.

In some embodiments of the second aspect, the single-domain antibody binds to the HLA-A02/HBsAg complex at an epitope comprising one or more residues at positions 348-357 of HBsAg as set forth in SEQ ID NO: 51. In some embodiments, the amino acid sequence at positions 348-357 of HBsAg as set forth in SEQ ID NO: 51 is GLSPTVWLSV (SEQ ID NO: 1).

In some embodiments of the second aspect, the single-domain antibody binds to the HLA-A02/HBsAg complex at an epitope comprising at least one of residues at positions 349, 351, 352, 353, 354, 355, 356, and 357 of HBsAg as set forth in SEQ ID NO: 51.

In some specific embodiments of the second aspect, the single-domain antibody binds to the HLA-A02/HBsAg complex at an epitope comprising residues at positions 349, 352, 353, 354, 355, 356, and 357 of HBsAg as set forth in SEQ ID NO: 51. In some embodiments, the amino acid residues at positions 349, 352, 353, 354, 355, 356, and 357 of HBsAg as set forth in SEQ ID NO: 51 correspond to the amino acid residues at positions 2, 5, 6, 7, 8, 9, and 10 of the HBsAg₃₄₈₋₃₅₇ (GLSPTVWLSV) polypeptide as set forth in SEQ ID NO: 1.

In some specific embodiments of the second aspect, the single-domain antibody binds to the HLA-A02/HBsAg complex at an epitope comprising residues at positions 351, 352, 353, 354, 355, 356, and 357 of HBsAg as set forth in SEQ ID NO: 51. In some embodiments, the amino acid residues at positions 351, 352, 353, 354, 355, 356, and 357 of HBsAg as set forth in SEQ ID NO: 51 correspond to the amino acid residues at positions 4, 5, 6, 7, 8, 9, and 10 of the HBsAg₃₄₈₋₃₅₇ (GLSPTVWLSV) polypeptide as set forth in SEQ ID NO: 1.

In some specific embodiments of the second aspect, the single-domain antibody binds to the HLA-A02/HBsAg complex at an epitope comprising residues at positions 349, 351, 352, 353, 354, 355, 356, and 357 of HBsAg as set forth in SEQ ID NO: 51. In some embodiments, the amino acid residues at positions 349, 351, 352, 353, 354, 355, 356, and 357 of HBsAg as set forth in SEQ ID NO: 51 correspond to the amino acid residues at positions 2, 4, 5, 6, 7, 8, 9, and 10 of the HBsAg₃₄₈₋₃₅₇ (GLSPTVWLSV) polypeptide as set forth in SEQ ID NO: 1.

In some embodiments of the second aspect, the single-domain antibody is in the form of a monovalent or multivalent single-domain antibody that binds to the HLA-A02/HBsAg complex.

In some embodiments of the second aspect, the single-domain antibody is a monovalent single-domain antibody that binds to the HLA-A02/HBsAg complex.

In some embodiments of the second aspect, the single-domain antibody is a bivalent single-domain antibody that binds to the HLA-A02/HBsAg complex.

In some embodiments of the second aspect, the single-domain antibody comprises two heavy chain variable regions from monovalent single-domain antibodies that bind to the HLA-A02/HBsAg complex.

In some embodiments of the second aspect, the single-domain antibody comprises two heavy chain variable regions from monovalent single-domain antibodies that bind to the HLA-A02/HBsAg complex, which are linked via direct fusion.

In some embodiments of the second aspect, the single-domain antibody comprises two heavy chain variable regions from monovalent single-domain antibodies that bind to the HLA-A02/HBsAg complex, which are linked via a linker. In some embodiments, the linker comprises a GS-type flexible peptide, such as (GGGGS)n, where n is an integer ≥ 1. In some specific embodiments, the linker is GGGGSGGGGSGGGGS (SEQ ID NO: 52).

In some embodiments of the second aspect, the single-domain antibody comprises the amino acid sequence as set forth in SEQ ID NO: 35.

In some embodiments of the second aspect, the single-domain antibody comprises the amino acid sequence as set forth in SEQ ID NO: 36.

In some embodiments of the second aspect, the single-domain antibody comprises the amino acid sequence as set forth in SEQ ID NO: 71.

In some embodiments of the second aspect, the single-domain antibody comprises the amino acid sequence as set forth in SEQ ID NO: 72.

In some embodiments of the second aspect, the single-domain antibody comprises an amino acid sequence that differs from the amino acid sequence as set forth in SEQ ID NOs: 35, 36, 71, or 72 by about 1, 2, 3, 4, 5, 6, 7, 8, 9, or 10 amino acid substitutions, deletions, and/or additions.

In some embodiments of the second aspect, the single-domain antibody comprises an amino acid sequence that has at least 90%, 91%, 92%, 93%, 94%, 95%, 96%, 97%, 98%, 99%, or higher identity to the amino acid sequence as set forth in SEQ ID NOs: 35, 36, 71, or 72.

In some embodiments of the second aspect, the amino acid sequence as set forth in SEQ ID NOs: 35, 36, 71, or 72 may also be truncated by about 1, 2, 3, 4, 5, 6, 7, 8, 9, 10, 11, 12, 13, 14, 15, 20, 25, or more amino acids at the C-terminus or N-terminus, while still retaining the function similar to that of the single-domain antibody.

In some embodiments of the second aspect, the amino acid sequence as set forth in SEQ ID NOs: 35, 36, 71, or 72 may also have 1, 2, 3, 4, 5, 6, 7, 8, 9, 10, 11, 12, 13, 14, 15, 20, 25, or more amino acids added at the C-terminus or N-terminus, while still retaining the function similar to that of the single-domain antibody.

In some embodiments of the second aspect, the amino acid sequence as set forth in SEQ ID NOs: 35, 36, 71, or 72 may also have 1, 2, 3, 4, 5, 6, 7, 8, 9, 10, 11, 12, 13, 14, 15, 20, 25, or more amino acids added or deleted at regions other than the C-terminus or the N-terminus, provided that the altered amino acid sequence substantially retains the function similar to that of the single-domain antibody.

In a third aspect, the present application provides a nucleic acid molecule encoding the bispecific antibody of the first aspect or the single-domain antibody of the second aspect.

In some embodiments of the third aspect, the nucleic acid molecule may include a DNA molecule and an RNA molecule. The nucleic acid molecule may be single-stranded or double-stranded and may be cDNA.

In some embodiments of the third aspect, the nucleic acid molecule is operably linked to a regulatory nucleotide sequence that can be recognized by a host cell transformed with a vector.

In a fourth aspect, the present application provides a pharmaceutical composition comprising the bispecific antibody of the first aspect or the single-domain antibody of the second aspect, and a pharmaceutically acceptable excipient, diluent, or carrier.

In some embodiments of the fourth aspect, the pharmaceutical composition is used for the prevention or treatment of a disease caused by HBV infection in a patient who is HLA-A02/HBsAg-positive.

In some embodiments of the fourth aspect, the disease caused by HBV infection is selected from the group consisting of hepatitis B, liver cirrhosis, liver fibrosis, and liver cancer.

In some embodiments of the fourth aspect, the pharmaceutical composition may further comprise one or more components selected from the group consisting of: a lubricant, such as talc, magnesium stearate, and mineral oil; a wetting agent; an emulsifier; a suspending agent; a preservative such as benzoic acid, sorbic acid and calcium propionate; a sweetener and/or a flavoring agent, and the like.

In some embodiments of the fourth aspect, the pharmaceutical composition of the present application may be formulated as a tablet, a pill, a powder, a lozenge, an elixir, a suspension, an emulsion, a solution, a syrup, a suppository, a capsule, and the like.

In some embodiments of the fourth aspect, the pharmaceutical composition of the present application may be delivered using any physiologically acceptable administration route which includes, but is not limited to, oral administration, parenteral administration, nasal administration, rectal administration, intraperitoneal administration, intravascular injection, subcutaneous administration, transdermal administration, inhalation administration, or the like.

In some embodiments of the fourth aspect, the pharmaceutical composition for therapeutic uses may be formulated for storage in the form of a lyophilized formulation or an aqueous solution by mixing a reagent of desired purity with a pharmaceutically acceptable carrier or excipient, as appropriate.

In a fifth aspect, the present application provides use of the bispecific antibody of the first aspect, the single-domain antibody of the second aspect, or the pharmaceutical composition of the fourth aspect in the manufacture of a medicament for the prevention or treatment of a disease caused by HBV infection in a patient who is HLA/HBsAg positive.

In some embodiments of the fifth aspect, the disease caused by HBV infection is selected from the group consisting of hepatitis B, liver cirrhosis, liver fibrosis, and liver cancer.

In a sixth aspect, the present application provides a method for preventing or treating a disease caused by HBV infection in a patient who is HLA-A02/HBsAg-positive, comprising administering to a subject in need thereof the bispecific antibody of the first aspect, the single-domain antibody of the second aspect, or the pharmaceutical composition of the fourth aspect.

In some embodiments of the sixth aspect, the disease caused by HBV infection is selected from the group consisting of hepatitis B, liver cirrhosis, liver fibrosis, and liver cancer.

In a seventh aspect, the present application provides use of the bispecific antibody of the first aspect or the single-domain antibody of the second aspect in the manufacture of a product for detecting HLA-A02/HBsAg-positive cells.

In some embodiments of the seventh aspect, the product is a kit, a test strip, a test card, or a microfluidic device.

In some embodiments of the seventh aspect, the product may further comprise detection labels, such as colloidal gold, chemiluminescent labels, fluorescent labels, nanoparticle labels, and the like.

In some embodiments of the seventh aspect, the product may further include other reagents for detection, such as an enzyme or colloidal gold labeled antigen or antibody, a substrate, a reference standard, a diluent, a wash buffer, and the like.

In some embodiments of the seventh aspect, the product may further comprise reagents for processing a biological sample for detection, and the biological sample may be blood.

In some embodiments of the seventh aspect, the product may further include instructions for use.

The present application further provides a vector comprising a nucleic acid molecule encoding the bispecific antibody of the first aspect, or the single-domain antibody of the second aspect, and a host cell comprising the nucleic acid molecule or the vector. In other aspects, the present application also provides a method for producing the bispecific antibody of the first aspect, or the single-domain antibody of the second aspect. In some embodiments, the method for producing the bispecific antibody of the first aspect or the single-domain antibody of the second aspect comprises culturing the host cell to facilitate expression of the nucleic acid molecule. In some embodiments, the method for producing the bispecific antibody of the first aspect, or the single-domain antibody of the second aspect, further comprises recovering the bispecific antibody or the single-domain antibody from the host cell culture medium.

It should be understood that the foregoing detailed description is intended only to enable those skilled in the art to better understand the present application and is not intended to limit it in any way. Various modifications and variations to the described embodiments can be made by those skilled in the art.

The following Examples are for purposes of illustration only and are not intended to limit the scope of the present application.

### Examples

### Example 1: Preparation of recombinant proteins

In the preparation of bispecific antibodies based on HLA-A02/HBsAg and CD3, a variety of recombinant proteins were utilized, including the extracellular domain of human CD3E (hCD3E, SEQ ID NO: 48) and the extracellular domain of human CD3D (hCD3D, SEQ ID NO: 49). Meanwhile, the following construct was prepared according to the Reference ^{[20]}: the disulfide trap single-chain trimer (dtSCT) structure of MHC class I complex presenting the HBsAg₃₄₈₋₃₅₇ (GLSPTVWLSV) antigen peptide, i.e., the HLA-A02 complex presenting GLSPTVWLSV (MIP-H1-01, SEQ ID NO: 50). These recombinant proteins all have various post-translational modifications (e.g., glycosylation or disulfide bonds), and thus a mammalian cell expression system was used to maintain their structure and function. To facilitate protein purification and monoclonal antibody functional identification, a His tag (His, SEQ ID NO: 54) or the Fc fragment of murine IgG2a antibody (mFc, SEQ ID NO: 55) was added to the C-terminus of these recombinant proteins. In the preparation of recombinant antibodies, the antibody heavy chain constant region can be the human IgG1 subtype (SEQ ID NO: 56) or its various mutants, such as IgG1H (SEQ ID NO: 57), IgG1K (SEQ ID NO: 58), IgG1m3-H (SEQ ID NO: 59), IgG1m3-K (SEQ ID NO: 60), IgG1m3-H1n1 (SEQ ID NO: 61), or IgG1m3-Kn1 (SEQ ID NO: 62); and the light chain constant region can be the human lambda (λ) isotype (SEQ ID NO: 63) or the human kappa (κ) isotype (SEQ ID NO: 64).

Genes including the His tag or mFc coding gene for the various recombinant proteins described above were designed and synthesized according to the amino acid sequences of the recombinant proteins of interest in the Uniprot database. The synthesized genes for the various recombinant proteins were cloned into a suitable vector for eukaryotic expression (such as pcDNA3.1, Invitrogen Inc.) using conventional techniques in molecular biology. Plasmids prepared for recombinant protein expression were then transfected using liposomes (such as 293fectin, Invitrogen Inc.) or other cationic transfection reagents such as PEI into HEK293 cells (such as HEK293F cells, Invitrogen Inc.), which were cultured in suspension under serum-free conditions for 3-4 days. The culture supernatant was then harvested by centrifugation or other methods.

Recombinant proteins expressed as fusions with His tags were subjected to one-step purification from the culture supernatant using a metal chelate affinity chromatography column (such as HisTrap FF from GE Inc.). Recombinant proteins expressed as fusions with mFc were subjected to one-step purification using a ProteinA/G affinity chromatography column (such as Mabselect SURE from GE Inc.). The preservation buffer for the recombinant proteins was then replaced with PBS (pH 7.0) or another suitable buffer using a desalting column (such as Hitrap Desalting, GE Inc.). If necessary, antibody samples may be sterilized by filtration, and then stored in aliquots at -20°C.

### Example 2: Screening of antibodies binding to the HLA-A02/HBsAg complex from a camel immune library

### 2.1 Construction of the camel immune library

One healthy adult bactrian camel was selected, and blood was collected to obtain baseline serum before immunization. For the first immunization, 1 mg of the MIP-H1-01 fusion protein was emulsified with Freund's complete adjuvant and injected subcutaneously at multiple sites. For booster immunizations every two weeks, 1 mg of the MIP-H1-01 fusion protein was emulsified with Freund's incomplete adjuvant and injected subcutaneously at multiple sites. A total of five booster immunizations were conducted, and blood samples were collected before each immunization for antibody titer analysis. For the seventh immunization, 1 mg of the MIP-H1-01 fusion protein without adjuvant was taken as an antigen and injected subcutaneously at multiple sites for a high-dose booster immunization. 200 mL of peripheral blood was collected for lymphocyte isolation after 3 days.

Camel peripheral blood lymphocytes were isolated using the Camel Peripheral Blood Lymphocyte Isolation Solution Kit (Solarbio, CAT#P5750). Total RNA was extracted from the isolated lymphocytes using a Total RNA Extraction Kit (TIANGEN Biotech (BEIJING) Co., Ltd., CAT#DP430). The extracted total RNA was used as a template to synthesize cDNA for the camel single-domain antibody heavy chain variable region (VHH) using a First-Strand cDNA Synthesis Kit (Thermo scientific, CAT#K1621). Gene-specific primers were used as primers for reverse transcription. The primers annealed to regions located in the heavy chain constant regions of antibodies, with the sequence of PCal-CH2R as: TCCTTCCCCGTCAGCCAGTCCT (SEQ ID NO: 53). The synthesized cDNAs were immediately stored at -70°C for future use. Then, the cDNA obtained by reverse transcription was used as a template to synthesize primers according to the Reference ^{[21]}, and the camel single-domain antibody VHH gene was amplified and isolated using nested PCR. Overlapping extension PCR technology was used to construct genes for single-chain variable fragments (scFvs). Finally, the amplified VHH genes were cloned into the vector pADSCFV-S (see Chinese Patent Application No. 201510097117.0 ^{[22]}) to construct a VHH library. The antibody library had a capacity of 1.41E+08, and an accuracy of 65.20%.

### 2.2 Screening of the camel immune library

The phage library displaying camel single-domain antibodies constructed in Section 2.1 was screened by a solid phase screening strategy (for the experimental protocol, see Phage Display: A Practical Approach, Clackson, T. (USA) and Lowman, H. B. (USA) (ed.), translated by Lan Ma et al., Chemical Industry Press, May 2008.5 ^{[23]}) using the recombinant MIP-H1-01 prepared in Example 1 as the antigen. Three rounds of screening were carried out by means of binding, elution, neutralization, infection and amplification. Finally, a TCR mimetic (TCRm) antibody N1B5 that specifically binds to MIP-H1-01 was obtained.

By using conventional molecular biological methods, the nucleotide sequence encoding the N1B5 heavy chain variable region (SEQ ID NO: 35) was cloned into a eukaryotic expression vector (such as pcDNA3.1, Invitrogen Inc.) containing the nucleotide sequence encoding the Fc region of the human IgG1 to express the N1B5-Fc recombinant protein. Meanwhile, the monoclonal antibody DP47-IgG1 of DP47 (germline gene antibody, see U.S. Patent Application No. US 20160200833A1 ^{[24]}, with the amino acid sequence of DP47VH as set forth in SEQ ID NO: 65 and the amino acid sequence of DP47Vκ as set forth in SEQ ID NO: 66) was prepared as a negative control. According to Patent Application WO2020193745 A1 ^{[25]}, the Vα (TCRA, SEQ ID NO: 67) and Vβ (TCRB, SEQ ID NO: 68) variable region genes of IMC-I109V TCR were synthesized and separately cloned into eukaryotic expression vectors containing the Fc fragment nucleotide sequence with a Knob-mutation fused to Cα and the Fc fragment nucleotide sequence with a Hole-mutation fused to Cβ. The resulting two chains, designated as H1-TCRA-Des-FcKn1 (SEQ ID NO: 69) and H1-TCRB-Des-G1m3-FcH1n1 (SEQ ID NO: 70), were co-expressed to assemble the TCR control molecule that binds to MIP-H1-01, named H1-TCR.

### Example 3: Identification of TCRm antibodies binding to the HLA-A02/HBsAg complex

### 3.1 Affinity analysis of anti-HLA-A02/HBsAg complex TCRm antibody

The affinity of the anti-HLA-A02/HBsAg TCRm antibody N1B5 was determined by surface plasmon resonance technique (SPR) using Biacore T200. Related reagents and consumables, such as the Amine Coupling Kit (BR-1000-50), the Human Antibody Capture Kit (BR-1008-39), a Series S CM5 chip (14100530), and 10×HBS-EP (BR100669) at pH 7.4, were purchased from GE Healthcare. According to the kit instructions, the surface of the carboxylated CM5 chip was activated with 1-ethyl-3-(3-dimethylaminopropyl) carbodiimide hydrochloride (EDC) and N-hydroxysuccinimide (NHS). The capture antibodies, anti-human IgG (Fc), were diluted to 25 µg/mL with 10 mM sodium acetate, pH 5.0, and then injected at a flow rate of 10 µL/min to achieve a coupling amount of approximately 10,000 response units (RU). This was followed by injection of 1 M ethanolamine to block unreacted groups. For the kinetic measurement, the TCRm antibody N1B5 was diluted to 1 µg/mL, and injected at 10 µL/min to ensure capture of approximately 70 RU by the anti-human Fc antibodies. MIP-H1-01 was then serially diluted to concentrations of 1.23 nM, 3.7 nM, 11.1 nM, 33.3 nM, and 100 nM, and injected at a flow rate of 30 µL/min from lowest to highest concentrations, with an association time of 90 seconds and a dissociation time of 1200 seconds. 3 M MgCl₂ solution was injected at a flow rate of 10 µL/min for 30 seconds to regenerate the chip surface. The association rate (Kₐ) and dissociation rate (K_{d}) were calculated by fitting the binding and dissociation sensorgrams with a 1:1 binding model using Biacore T200 Evaluation Software (version 3.2.1). The dissociation equilibrium constant (K_{D}) was calculated as the ratio K_{d}/Kₐ. The results are shown in Table 1.

**Table 1. Affinity of anti-HLA-A02/HBsAg complex TCRm antibody N1B5 for MIP-H1-01**

| | Kₐ(M⁻¹s⁻¹) | K_{d}(s⁻¹) | K_{D}(M) |
|---|---|---|---|
| N1B5 | 6.921E+5 | 9.696E-5 | 1.401E-10 |

### 3.2 Binding of anti-HLA-A02/HBsAg complex TCRm antibody to recombinant protein MIP-H1-01

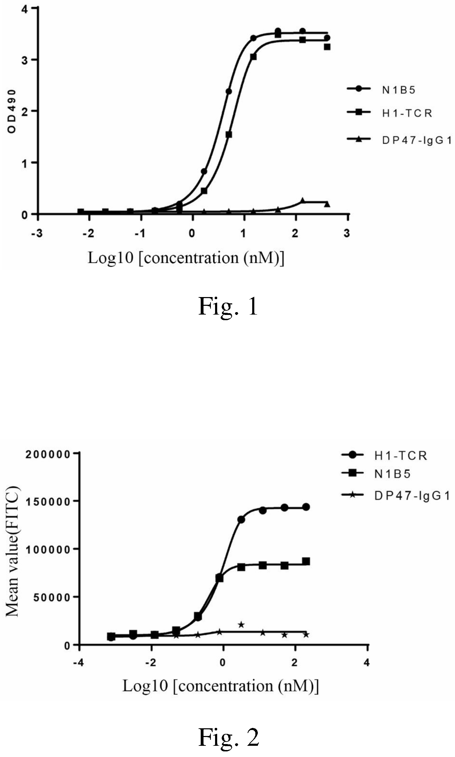

The anti-HLA-A02/HBsAg complex TCRm antibody N1B5 was coated on a 96-well ELISA plate at 5 µg/mL, 100 µL per well, and incubated overnight at 4°C. After blocking with the blocking solution (PBS containing 0.1% Tween-20 and 3% skim milk) at 37°C for 1 hour, the recombinant protein MIP-H1-01 was serially diluted with PBS at a starting concentration of 10 µg/mL (with 3-fold serial dilutions, for a total of 11 concentrations). 100 µL of of each dilution was added into each well of the blocked 96-well ELISA plate and incubated at 37°C for 1 hour. The ELISA plate was washed with PBS containing 0.1% Tween-20 and then 100 µL of HRP-conjugated Anti-His-Tag Mouse Monoclonal Antibody (Beijing CoWin Biotech Co., Ltd, CW0285M) was added and incubated at 37°C for 1 hour. The ELISA plate was washed with PBS containing 0.1% Tween-20, and OPD substrate chromogenic solution was added. After 5-10 minutes, the color reaction was terminated with 1 M H₂SO₄, and optical density values were measured using a microplate reader at dual wavelengths of 492 nm/630 nm. FIG. 1 shows the results demonstrating that N1B5 specifically binds to MIP-H1-01, indicating that N1B5 specifically binds to the HLA-A02/HBsAg complex.

### 3.3 Binding of anti-HLA-A02/HBsAg complex TCRm antibody to antigen peptide-pulsed T2 cells

T2 cells (human lymphoma cells, HLA-A02⁺, purchased from Shanghai Honsun Biological Technology Co., Ltd) were harvested, centrifuged, and resuspended in growth medium to a concentration of 1×10⁶ cells/mL. Then, 1 mL of the suspension was plated into each well of a 24-well plate. The HBsAg₃₄₈₋₃₅₇ (GLSPTVWLSV) peptide was chemically synthesized (GenScript Biotechnology Co., Ltd) and added to the 24-well plate at a final concentration of 50 µg/mL for overnight pulsing. After 16 hours, the cells were centrifuged and resuspended in PBS buffer containing 1% BSA to a concentration of 2×10⁶ cells/mL. 100 µL of the suspension was plated into each well of a 96-well V-bottom plate, and the supernatant was removed after centrifugation. The antibody N1B5, the positive control sample H1-TCR, and the negative control DP47-IgG1 were prepared by diluting with PBS to a starting concentration of 200 nM, with 4-fold serial dilutions, for a total of 10 concentrations. 100 µL of each dilution was added to each well containing cells and incubated at 4°C for 1 hour. After washing the wells three times with 200 µL PBS, the cells were incubated with Goat Anti-human IgG-FITC (Beijing Zhong Shan - Golden Bridge Biological Technology CO., LTD, ZF-0308) at 100 µL per well for 30 minutes at 4°C in the dark. After washing the wells three times with 200 µL PBS, the cells were resuspended in 100 µL PBS and detected in the FITC channel by flow cytometry (ACEA, Novocyte). FIG. 2 shows the results demonstrating that N1B5 specifically binds to the antigen peptide HBsAg₃₄₈₋₃₅₇-pulsed T2 cells, indicating that N1B5 binds to the HLA-A02/HBsAg complex on the cell surface.

### 3.4 Binding of anti-HLA-A02/HBsAg complex TCRm antibody to key amino acids of HBsAg

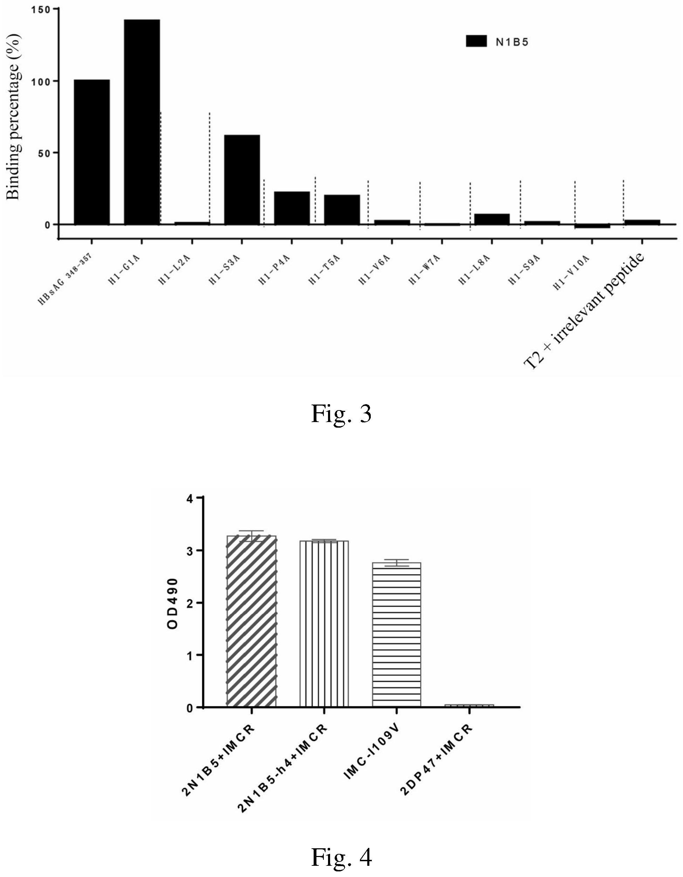

The alanine-scanning mutant peptides of HBsAg₃₄₈₋₃₅₇ (GLSPTVWLSV) were chemically synthesized. Following the method described in Section 3.3, the binding of N1B5 to alanine-scanning mutant peptides of HBsAg₃₄₈₋₃₅₇ (GLSPTVWLSV)-pulsed T2 cells was detected. The amino acid sequence of the irrelevant peptide is set forth in SEQ ID NO: 87. The binding of N1B5 to the alanine-scanning mutant peptides was calculated relative to that of the original peptide. FIG. 3 shows that the binding capacity of the TCRm antibody N1B5 is reduced by more than 70% after alanine substitution at positions 2, 4, 5, 6, 7, 8, 9, or 10 of HBsAg₃₄₈₋₃₅₇ (GLSPTVWLSV), indicating that residues 2, 4, 5, 6, 7, 8, 9, and 10 of HBsAg₃₄₈₋₃₅₇ (GLSPTVWLSV) are key amino acids for N1B5 binding.

### Example 4: Humanization and identification of anti-HLA-A02/HBsAg complex TCRm antibody

### 4.1 Humanization of anti-HLA-A02/HBsAg complex TCRm antibody

The TCRm antibody N1B5 was humanized to reduce its immunogenicity. A classic framework grafting strategy ^{[26]} was used for the humanization scheme. The amino acid sequence of N1B5 was compared to those of human antibody germline gene sequences in the IMGT database, respectively. Appropriate germline gene sequences were selected to provide framework regions 1 to 3 (FR1+FR2+FR3) of the antibody, and an appropriate J region gene sequence was selected to provide the framework region 4 (FR4). This template may be selected based on a variety of factors, such as the relative total length of the antibody, the size of the CDR, the amino acid residues at the junction between the antibody framework regions (FRs) and the hypervariable regions (CDRs), the overall sequence homology, and the like. The selected template may be a mixture of multiple sequences or a consensus template, with the aim of maintaining the appropriate conformation of the complementarity determining regions (CDRs) of the parent antibody as much as possible. At the same time, considering the solubility, stability, and expression yield of the humanized antibody, the four hotspot amino acids (37F/44E/45R/47F) in FR2 were back-mutated, respectively, and the humanized molecule N1B5-h4 was finally obtained.

By using conventional molecular biological methods, the nucleotide sequence encoding the N1B5-h4 variable region (SEQ ID NO: 36) was cloned into a eukaryotic expression vector containing the nucleotide sequence encoding the Fc region of the human IgG1 (such as pcDNA3.1, Invitrogen Inc.) to express the N1B5-h4-Fc recombinant protein.

### 4.2 Affinity analysis of humanized anti-HLA-A02/HBsAg complex TCRm antibody molecule

Following the method described in Section 3.1, the affinity of the recombinant anti-HLA-A02/HBsAg complex TCRm antibody N1B5-h4 was assayed using Biacore T200. The results are shown in Table 2.

**Table 2. Affinity of anti-HLA-A02/HBsAg complex TCRm antibody N1B5-h4 for MIP-H1-01**

| | Kₐ(M⁻¹s⁻¹) | K_{d}(s⁻¹) | K_{D}(M) |
|---|---|---|---|
| N1B5-h4 | 6.556E+5 | 1.252E-4 | 1.910E-10 |

### Example 5: Preparation and identification of TCRm×CD3 bispecific antibody

### 5.1 Preparation of TCRm×CD3 bispecific antibody

The nucleotide sequences encoding the heavy chain variable regions of the divalent N1B5 or divalent N1B5-h4 single-domain antibody against the anti-HLA-A02/HBsAg complex and the nucleotide sequence encoding the anti-CD3 antibody (IMCR-anti-CD3-scFv, see anti-CD3 v9 in U.S. Patent No. US 582133A ^{[27]}) were separately cloned into a suitable eukaryotic expression vector to co-express the bispecific antibody against both the HLA-A02/HBsAg complex and CD3. Sepcifically, the nucleotide sequence encoding 2N1B5 (SEQ ID NO: 71) or 2N1B5-h4 (SEQ ID NO: 72) was separately cloned into a eukaryotic expression vector fused with the nucleotide sequence encoding the Fc fragment with the Hole mutation (IgG1m3-FcH1n1, SEQ ID NO: 73), and the nucleotide sequence encoding IMCR-anti-CD3-ScFv (SEQ ID NO: 74) was cloned into a eukaryotic expression vector fused with the nucleotide sequence encoding the Fc fragment with the Knob mutation (IgG1m3-FcKn1, SEQ ID NO: 75). Meanwhile, a DP47 bispecific antibody 2DP47+IMCR based on the same structure was prepared as a negative control.

The eukaryotic expression vectors constructed for expressing 2N1B5-IgG1m3-FcH1n1 (SEQ ID NO: 45) or 2N1B5-h4-IgG1m3-FcH1n1 (SEQ ID NO: 46) and the eukaryotic expression vector for IMCR-anti-CD3-ScFv-IgG1m3-FcKn1 (SEQ ID NO: 47) were co-transfected into HEK293F cells using Lipofectamine and cultured in serum-free medium for 3-5 days. The culture supernatant was collected by centrifugation and the bispecific antibody was purified from the culture supernatant using Protein A affinity chromatography (such as Mabselect SURE, GE Healthcare.). Then, the recombinant protein preservation buffer was exchanged with PBS (pH 7.0) or another suitable buffer using a desalting column (such as Hitrap Desalting, GE Healthcare). The desalted protein solution was further purified by size-exclusion chromatography (SEC, Superdex200, GE Healthcare) to obtain the protein of interest. If necessary, the antibody samples were sterilized by filtration and stored in aliquots at -20°C for further use.

Preparation of the bifunctional molecule IMC-I109V based on TCR and CD3. The single-chain variable fragment IMCR-anti-CD3-scFv was fused to the N-terminus of the H1-TCRB-Des-G1m3-FcH1n1 chain, generating IMC-I109V, consisting of two chains, named H1-TCRA-Des-G1m3-FcKn1 (SEQ ID NO: 69) and IMCR-anti-CD3-scFv-TCRB-Des-G1m3-FcH1n1 (SEQ ID NO: 76), respectively. The expression and purification of IMC-I109V followed the same method as that for the bispecific antibodies 2N1B5+IMCR and 2N1B5-h4+IMCR. The final samples were sterilized by filtration and stored in aliquots at -20°C for further use.

### 5.2 Affinity analysis of TCRm×CD3 bispecific antibodies

Following the method described in Section 3.1, the affinities of the recombinant TCRm×CD3 bispecific antibodies 2N1B5+IMCR and 2N1B5-h4+IMCR were assayed using Biacore T200. The results are shown in Tables 3 and 4.

**Table 3. Affinity of TCRm×CD3 bispecific antibody for MIP-H1-01**

| | Kₐ(M⁻¹s⁻¹) | K_{d}(s⁻¹) | K_{D}(M) |
|---|---|---|---|
| 2N1B5+IMCR | 6.132E+5 | 1.452E-4 | 2.367E-10 |
| 2N1B5-h4+IMCR | 2.629E+5 | 1.397E-4 | 5.314E-10 |
| IMC-I109V | 5.241E+4 | 8.693E-5 | 1.659E-9 |

**Table 4. Affinity of TCRm×CD3 bispecific antibody for CD3D-CD3E-mFc**

| | Kₐ(M⁻¹s⁻¹) | K_{d}(s⁻¹) | K_{D}(M) |
|---|---|---|---|
| 2N1B5+IMCR | 5.918E+4 | 1.753E-4 | 2.962E-9 |
| 2N1B5-h4+IMCR | 6.469E+4 | 1.32E-4 | 2.041E-9 |
| IMC-I109V | 7.019E+4 | 2.591E-4 | 3.692E-9 |

### 5.3 TCRm×CD3 bispecific antibody simultaneously recognizes both antigens MIP-H1-01 and CD3

A conventional ELISA method was used to detect that the TCRm×CD3 bispecific antibody simultaneously binds to the two directional antigens CD3 and MIP-H1-01.

The antigen CD3D-CD3E-mFc was coated on a 96-well ELISA plate (3 µg/mL, 100 µL per well) and incubated overnight at 4°C. Then, the wells were blocked with blocking solution (PBS containing 0.1% Tween-20 and 3% skim milk) at 37°C for 1 hour. TCRm×CD3 bispecific antibodies were serially diluted with PBS to a starting concentration of 10 µg/mL. 100 µL of each dilution was added into each well of the blocked 96-well ELISA plate and incubated at 37°C for 1 hour. The ELISA plate was washed with PBS containing 0.1% Tween-20, and then MIP-H1-01 antigen (10 µg/mL) was added at 100 µL per well and incubated at 37°C for 1 hour. The ELISA plate was washed with PBS containing 0.1% Tween-20, and then HRP-Conjugated Anti-His-Tag Mouse Monoclonal Antibody (Beijing CoWin Biotech Co., Ltd, CW0285M) was added and incubated at 37°C for 1 hour. The ELISA plate was washed with PBS containing 0.1% Tween-20, and OPD substrate chromogenic solution was added. After 5-10 minutes, the color reaction was terminated with 1 M of H₂SO₄, and optical density values were measured using a microplate reader at dual wavelengths of 492 nm/630 nm. The ELISA analysis results shown in FIG. 4 demonstrate that the TCRm×CD3 bispecific antibodies 2N1B5+IMCR and 2N1B5-h4+IMCR can simultaneously recognize the bidirectional antigens CD3 and MIP-H1-01.

### 5.4 TCRm×CD3 bispecific antibody mediates the activation of Jurkat-Dual cells

### 5.4.1 TCRm×CD3 bispecific antibody mediates the activation of Jurkat-Dual cells by targeting antigen peptide-pulsed T2 cells

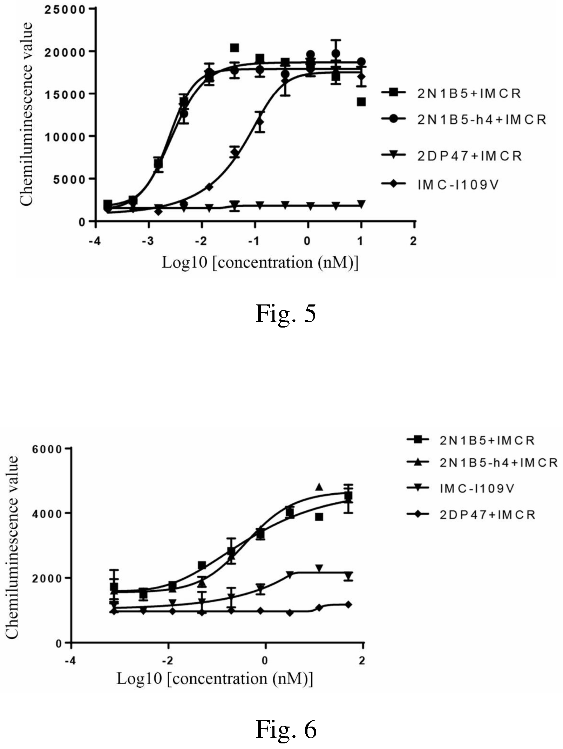

Log-phase T2 cells were harvested, centrifuged, and resuspended in growth medium to a concentration of 1×10⁶ cells/mL. Then, 1 mL of the suspension was plated into each well of a 24-well plate. The HBsAg₃₄₈₋₃₅₇ (GLSPTVWLSV) peptide was added to the 24-well plate at a final concentration of 50 µg/mL for overnight pulsing. After 16 hours, the cells were centrifuged again and resuspended in RPMI 1640 medium to a concentration of 4×10⁵ cells/mL. Then, 50 µL of the suspension was plated into each well of a cell plate. Log-phase Jurkat-Dual cells (Invivogen) were harvested, centrifuged, and resuspended in RPMI1640 medium to a concentration of 4×10⁵ cells/mL. Then, 50 µL of the suspension was added into each well of the T2-seeded plate to obtain a final effector-to-target (E: T) ratio of 1:1. The bispecific antibodies 2N1B5+IMCR and 2N1B5-h4+IMCR, at a starting concentration of 10 nM (with 3-fold serial dilutions, for a total of 11 concentrations) were then added at 100 µL per well. The negative control sample 2DP47+IMCR and the positive control sample IMC-I109V were used at the same concentrations. After 24 hours of incubation, the supernatant was collected, and the ability of the TCRm×CD3 bispecific antibodies to activate Jurkat-Dual cells by targeting the antigen peptide-pulsed T2 cells was assayed according to the QUANTI-Luc^{™} instructions (Invivogen, CAT#rep-qlc2). The results shown in FIG. 5 demonstrate that the bispecific antibodies 2N1B5+IMCR and 2N1B5-h4+IMCR can specifically mediate the activation of Jurkat-Dual cells by targeting the antigen peptide-pulsed T2 cells. Furthermore, compared with IMC-I109V, the TCRm×CD3 bispecific antibodies 2N1B5+IMCR and 2N1B5-h4+IMCR exhibit stronger activation effects on Jurkat-Dual cells. The EC₅₀ values are shown in Table 5.

**Table 5. EC₅₀ value of TCRm×CD3 bispecific antibody in mediating activation of Jurkat-Dual cells by targeting antigen peptide-pulsed T2 cells**

| | 2N1B5+IMCR | 2N1B5-h4+IMCR | IMC-I109V |
|---|---|---|---|
| EC₅₀(nM) | 0.002413 | 0.002758 | 0.06414 |

### 5.4.2 TCRm×CD3 bispecific antibody mediates the activation of Jurkat-Dual cells by targeting HLA-A02⁺/HBsAg⁺ tumor cells

HepG2 cells (human liver cancer cells, Cell Resource Center, Institute of Basic Medical Sciences) were HLA-A02 positive cells. A cell strain, HepG2-HBsAg, stably expressing HBsAg (hepatitis B surface antigen), was constructed based on HepG2 cells. Log-phase HepG2-HBsAg cells were harvested, centrifuged, and resuspended in RPMI 1640 medium to a concentration of 4×10⁵ cells/mL. Then, 50 µL of the suspension was plated into each well of a cell plate. Log-phase Jurkat-Dual cells were harvested, digested, centrifuged and resuspended in RPMI 1640 medium to a concentration of 4×10⁵ cells/mL. Then, 50 µL per well of the suspension was added into each well of the cell plate to obtain a final E:T ratio of 1:1. The bispecific antibodies 2N1B5+IMCR and 2N1B5-h4+IMCR at a starting concentration of 50 nM (with 4-fold serial dilutions, for a total of 10 concentrations), were then added at 100 µL per well. The negative control sample 2DP47+IMCR and the positive control sample IMC-I109V were used at the same concentrations. After 48 hours of incubation, the supernatant was collected, and the ability of the TCRm×CD3 bispecific antibodies to mediate the specific activation of Jurkat-Dual cells by targeting HLA-A02⁺/HBsAg⁺ cells was assayed according to the QUANTI-Luc^{™} instructions. The results shown in FIG. 6 demonstrate that the bispecific antibodies 2N1B5+IMCR and 2N1B5-h4+IMCR can specifically mediate the activation of Jurkat-Dual cells by targeting the HLA-A02⁺/HBsAg⁺ tumor cells HepG2-HBsAg. Furthermore, compared with IMC-I109V, the TCRm×CD3 bispecific antibodies 2N1B5+IMCR and 2N1B5-h4+IMCR exhibit stronger activation effects on Jurkat-Dual cells. The EC₅₀ values are shown in Table 6.

**Table 6. EC₅₀ value of TCRm×CD3 bispecific antibody in mediating activation of Jurkat-Dual cells by targeting HLA-A02⁺/HBsAg⁺ tumor cells**

| | 2N1B5+IMCR | 2N1B5-h4+IMCR | IMC-I109V |
|---|---|---|---|
| EC₅₀(nM) | 0.3894 | 0.4088 | 0.6211 |

### 5.5 TCRm×CD3 bispecific antibody mediates specific killing of target cells by PBMCs

### 5.5.1 Isolation of human peripheral blood mononuclear cells (PBMCs)

Blood was collected from healthy volunteers (50 mL from each) who had signed informed consent. The inclusion criteria for volunteers were: age over 18 years; no HIV and HBV infection; normal results of blood routine test; and non-pregnant and non-lactating female.

PBMCs were isolated from the whole blood of the volunteers by Ficoll density gradient centrifugation and cultured in RPMI 1640 medium.

### 5.5.2 TCRm×CD3 bispecific antibody mediates killing of antigen peptide-pulsed T2 cells by PBMCs

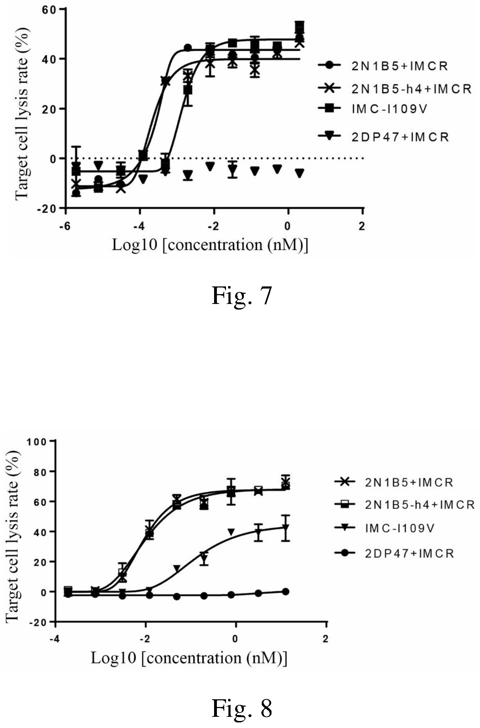

Log-phase T2 cells were harvested, centrifuged, and resuspended in growth medium to a concentration of 1×10⁶ cells/mL. Then, 1 mL of the suspension was plated into each well of a 24-well plate. The HBsAg₃₄₈₋₃₅₇(GLSPTVWLSV) peptide was added to the 24-well plate at a final concentration of 50 µg/mL for overnight pulsing. After 16 hours, the cells were centrifuged and resuspended in RPMI 1640 medium to a concentration of 4×10⁵ cells/mL. Then, 50 µL of the suspension was plated into each well of a 96-well cell plate. The bispecific antibodies 2N1B5+IMCR and 2N1B5-h4+IMCR, at a starting concentration of 2 nM (with 4-fold serial dilutions, for a total of 11 concentrations), were then added at 100 µL per well. The negative control sample 2DP47+IMCR and the positive control sample IMC-I109V were used at the same concentrations. PBMCs (effector cells) were resuspended to 4×10⁶ cells/mL. Then, 50 µL of the suspension was added into each well of the cell plate, achieving a final E: T ratio of 10:1. A separate target cell control (antigen peptide-pulsed T2 cells), a separate effector cell control (PBMCs), and a medium-only blank control were simultaneously prepared. The volume of each well was adjusted to 200 µL with medium. After 24 hours of incubation, the supernatant was collected, and the TCRm×CD3 bispecific antibody-mediated killing of the antigen peptide-pulsed T2 cells by PBMCs was assayed according to the CytoTox96^{®} Non-Radioactive Cytotoxicity Assay reagent instructions (Promega, CAT#G1780). The results show that the TCRm×CD3 bispecific antibodies 2N1B5+IMCR and 2N1B5-h4+IMCR can mediate specific killing of the antigen peptide-pulsed T2 cells by PBMCs. Compared with IMC-I109V, the bispecific antibodies 2N1B5+IMCR and 2N1B5-h4+IMCR exhibit higher activity in mediating killing of the antigen peptide-pulsed T2 cells by PBMCs, as shown in Table 7 and FIG. 7.

**Table 7. EC₅₀ value of TCRm×CD3 bispecific antibody in mediating killing of antigen peptide-pulsed T2 cells by PBMCs**

| | 2N1B5+IMCR | 2N1B5-h4+IMCR | IMC-I109V |
|---|---|---|---|
| EC₅₀ (nM) | 0.000278 | 0.0002095 | 0.001455 |

### 5.5.3 TCRm×CD3 bispecific antibody mediates killing of HLA-A02⁺/HBsAg⁺ cells by PBMCs

Log-phase HepG2-HBsAg cells were harvested, digested, centrifuged, and resuspended in RPMI 1640 medium to a concentration of 4×10⁵ cells/mL. Then, 50 µL of the suspension was plated into each well of a cell plate. The bispecific antibodies 2N1B5+IMCR and 2N1B5-h4+IMCR, at a starting concentration of 12.5 nM (with 4-fold serial dilutions, for a total of 9 concentrations), were then added at 100 µL per well. The negative control sample 2DP47+IMCR and the positive control sample IMC-I109V were used at the same concentrations. PBMCs (effector cells) were resuspended to 4×10⁶ cells/mL. Then, 50 µL of the suspension was added into each well of the cell plate, achieving a final E: T ratio of 10:1. A separate target cell control, a separate effector cell control (PBMCs), and a medium-only blank control were simultaneously prepared. The volume of each well was adjusted to 200 µL with medium. After 48 hours of incubation, the supernatant was collected, and the TCRm×CD3 bispecific antibody-mediated killing of HLA-A02⁺/HBsAg⁺ cells by PBMCs was assayed according to the CytoTox96^{®} Non-Radioactive Cytotoxicity Assay reagent instructions (Promega, G1780). The results show that 2N1B5+IMCR and 2N1B5-h4+IMCR can mediate specific killing of HLA-A02⁺/HBsAg⁺ cells by PBMCs and exhibit higher killing activity than IMC-I109V (FIG. 8 and Table 8).

**Table 8. EC₅₀ value of TCRm×CD3 bispecific antibody in mediating killing of HepG2-HBsAg cells by PBMCs**

| | 2N1B5+IMCR | 2N1B5-h4+IMCR | IMC-I109V |
|---|---|---|---|
| EC₅₀ (nM) | 0.008834 | 0.00952 | 0.1273 |

### Example 6: Analysis of key amino acid in HBsAg₃₄₈₋₃₅₇ (GLSPTVWLSV) recognized by TCRm×CD3 bispecific antibody

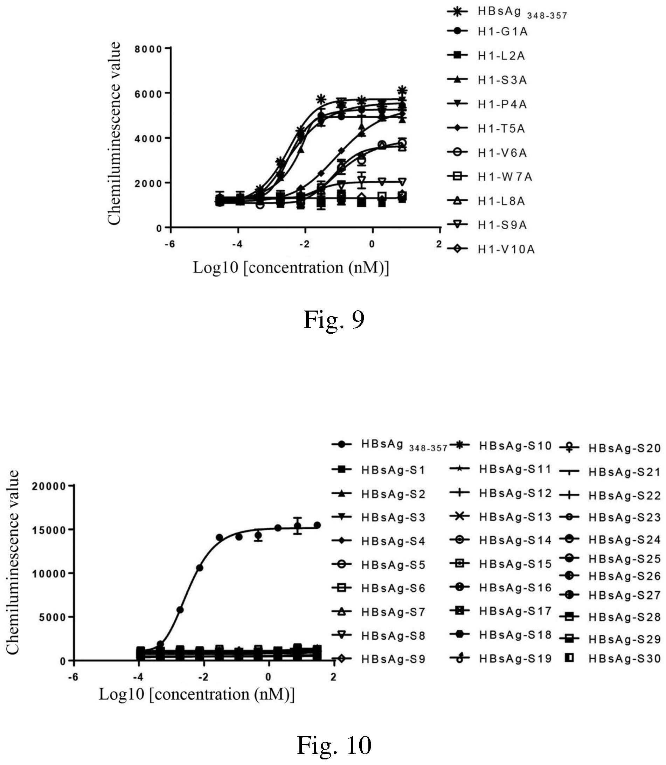

The alanine-scanning mutant peptides of HBsAg₃₄₈₋₃₅₇ (GLSPTVWLSV) were chemically synthesized. Following the method described in Section 5.4.1, the specific activation of Jurkat-Dual cells by targeting the alanine-scanning peptide-pulsed T2 cells was assayed. The results show that 2N1B5-h4+IMCR cannot mediate the activation of Jurkat-Dual cells by targeting alanine-scanning mutant peptides (at positions 2, 5, 6, 7, 8, 9, and 10)-pulsed T2 cells (FIG. 9), indicating that the amino acids at positions 2, 5, 6, 7, 8, 9, and 10 of HBsAg₃₄₈₋₃₅₇ (GLSPTVWLSV) are the key amino acids for 2N1B5-h4+IMCR binding.

### Example 7: Interaction analysis of TCRm×CD3 bispecific antibody with similar peptides

The similar peptides of HBsAg₃₄₈₋₃₅₇ (GLSPTVWLSV) listed in Table 9 were chemically synthesized.

**Table 9. Information on similar peptides of HBsAg₃₄₈₋₃₅₇ (GLSPTVWLSV)**

| **SEQ ID NO:** | **Name of peptide** | **Sequence** | **Uniprot ID** | **Normal tissues presenting peptide** |
|---|---|---|---|---|
| 1 | HBsAg₃₄₈₋₃₅₇ | GLSPTVWLSV | P17399 | |
| 2 | HBsAg-S1 | LMAEMGVHSV | Q9NWZ5-1 | adrenal gland, cerebellum, kidney, lung, |
| 3 | HBsAg-S2 | KLLEPVPVSV | Q86W34 | adrenal gland, bone marrow, brain, |
| 4 | HBsAg-S3 | GLWHGILTSV | Q9Y2E4 | kidney |
| 5 | HBsAg-S4 | GLVGNPLPSV | Q9H583 | Lung, kidney |
| 6 | HBsAg-S5 | VLEDGPWKTV | Q96SQ7 | lung, ovary, small intestine, thyroid gland, |
| 7 | HBsAg-S6 | ALLGVWTSV | Q12797 | ovary |
| 8 | HBsAg-S7 | FLPQPVPLSV | Q86T82 | |
| 9 | HBsAg-S8 | GLINTGVLSV | Q8NB78 | |
| 10 | HBsAg-S9 | ALSPITKLSV | Q8WUM0 | |
| 11 | HBsAg-S10 | GLSPLRPPSV | O14686 | |
| 12 | HBsAg-S11 | GLSEEKPLSV | Q9NX61 | |
| 13 | HBsAg-S12 | GLSPVTNLTV | P30304 | |
| 14 | HBsAg-S13 | SGLSPTVAVL | O60443 | |
| 15 | HBsAg-S14 | SLSPTVWFL | Q99500 | |
| 16 | HBsAg-S15 | AQPTVWLTI | A0A5K1VW70 | |
| 17 | HBsAg-S16 | HLLSTPWLQL | P05181 | liver |
| 18 | HBsAg-S17 | ILVDTVWAL | O00505 | cerebellum, lung, ovary, spleen, thymus, tongue |
| 19 | HBsAg-S18 | KLLETKWTL | P08729 | lung, pancreas |
| 20 | HBsAg-S19 | RVPDFYFHTI | Q5XKL5 | brain, kidney, small intestine |
| 21 | HBsAg-S20 | SLAETFWETT | P29144 | - |
| 22 | HBsAg-S21 | YLPHTFWITL | Q9P241 | - |
| 23 | HBsAg-S22 | GLWHGVLTSV | Q14689 | |
| 24 | HBsAg-S23 | SLAERLFFQV | Q8N9F0 | cerebellum |
| 25 | HBsAg-S24 | WLQDFVPTSV | Q9Y2D2-1 | |
| 26 | HBsAg-S25 | WLLPGRWTSV | Q9H6A9 | |
| 27 | HBsAg-S26 | LLLATVLQAV | O60613 | |
| 28 | HBsAg-S27 | YLKDGPYITA | Q6P2Q9 | ovary |
| 29 | HBsAg-S28 | GLMDTVKKV | Q9Y692 | cerebellum, kidney, lung, ovary, spleen |
| 30 | HBsAg-S29 | ALDSGAFQSV | Q8NFW8 | adrenal gland, bone marrow, kidney |
| 31 | HBsAg-S30 | FLWDEGFHQL | Q13724 | adrenal gland, bone marrow, cerebellum, kidney, liver, lung, ovary, small intestine, |

Following the method described in Section 5.4.1, the specific activation of Jurkat-Dual cells by targeting different similar peptides-pulsed T2 cells was assayed. The results shown in FIG. 10 demonstrate that 2N1B5-h4+IMCR can only specifically mediate the activation of Jurkat-Dual cells by targeting the HBsAg₃₄₈₋₃₅₇ (GLSPTVWLSV) peptide-pulsed T2 cells, but cannot mediate the activation of Jurkat-Dual cells by targeting other similar peptides-pulsed T2 cells.

### Example 8: Interaction analysis of TCRm×CD3 bispecific antibody with different antigen-negative tumor cells

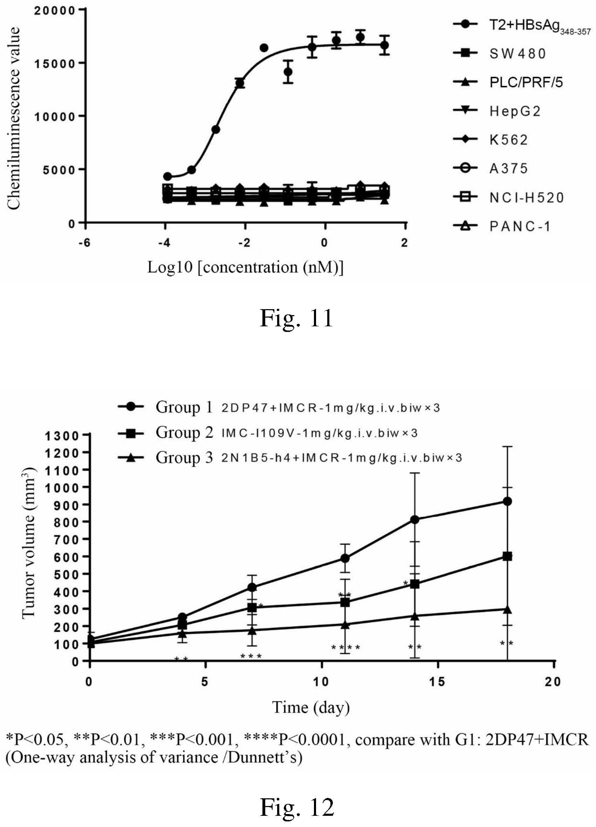

Different tumor cells were collected, and their antigen information is shown in Table 10. Following the method described in Sections 5.4.1 and 5.4.2, the ability of the bispecific antibody 2N1B5-h4+IMCR to mediate specific activation of Jurkat-Dual cells by targeting tumor cells was assayed. The results showed that 2N1B5-h4+IMCR can only specifically mediate the activation of Jurkat-Dual by targeting HLA-A02⁺/HBsAg⁺ cells, but cannot mediate the activation of Jurkat-Dual cells by targeting antigen-negative cells (FIG. 11).

**Table 10. Information on antigen expression in different cells**

| Cell | HLA-A02 | HBsAg | Cell | HLA-A02 | HBsAg |
|---|---|---|---|---|---|
| T2+HBsAg₃₄₈₋₃₅₇ | + | + | SW480 | + | - |
| PANC-1 | + | - | K562 | - | - |
| HepG2 | + | - | NCI-H520 | - | - |
| A-375 | + | - | PLC/PRF/5 | - | + |

| | | | | | |
|---|---|---|---|---|---|
| Note: "+" means positive and "-" means negative. | | | | | |

### Example 9: Antitumor effect of TCRm×CD3 bispecific antibody in hPBMC immune reconstitution mice

PBMCs were obtained by sorting following the method described in Section 5.5.1.

Thirty female B-NDG mice aged 5-6 weeks (Biocytogen Pharmaceuticals (Beijing) Co., Ltd.) were selected. Each mouse was inoculated subcutaneously on the right flank with 1×10⁷ HepG2-HBsAg cells, and inoculated intraperitoneally with 8×10⁶ human PBMCs on the next day. When the average tumor volume reached 90 mm³, the mice were randomly assigned to three groups based on tumor volume: the bispecific antibody 2N1B5-h4+IMCR group, the negative control 2DP47+IMCR group and the positive control IMC-I109V group, with 7 mice in each group. The day of grouping was defined as day 0, and administration was performed. The mice were administered via tail vein injection at a dose of 1 mg/kg, twice a week for six times, with continuous observation. Efficacy was evaluated according to the tumor growth inhibition (TGI).

The animals generally maintained a good mental state throughout the experiment. The bispecific antibody 2N1B5-h4+IMCR at a dose of 1 mg/kg (Group 3) had a significant inhibitory effect on tumor growth, with a relative TGI (%) of 76.01% on day 11, which showed a highly significant difference compared to the negative control group (Group 1) (p<0.0001). The bispecific antibody 2N1B5-h4+IMCR can effectively inhibit the growth of HLA-A02⁺/HBsAg⁺ tumors, and showed superior efficacy compared to IMC-I109V (Group 2) (FIG. 12). No animals in the treated groups died, and no significant drug toxicity was observed.

### Example 10: Binding site analysis of TCRm antibody N1B5-h4 to HBsAg₃₄₈₋₃₅₇ peptide in HLA-A02/HBsAg complex

To study the binding sites of the TCRm antibody N1B5-h4 to the HBsAg₃₄₈₋₃₅₇ peptide in the HLA-A02/HBsAg complex, the recombinant protein N1B5-h4-Twin-Strep-tag (SEQ ID NO: 88) and recombinant protein MIP-H1-01 were prepared using HEK293 cells. The two proteins were co-incubated at a molecular ratio of 1:1 to form the antigen-antibody complex N1B5-h4/MIP-H1-01, which was crystallized under conditions of 0.2 M calcium chloride, 0.1 M HEPES, and 28% PEG400. X-ray diffraction experiments were performed on the complex crystals, and data were collected at a resolution of 2.87Å. The crystal structure of the N1B5-h4/MIP-H1-01 antigen-antibody complex was resolved using molecular replacement.

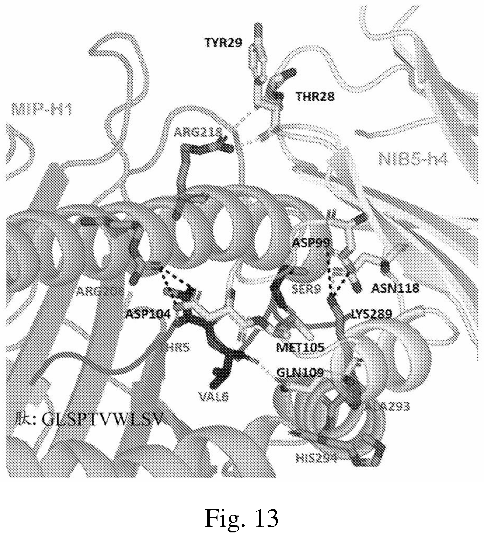

The interaction area between N1B5-h4 and MIP-H1-01 is approximately 825.5Å2, involving hydrogen bonds, salt bridges, van der Waals interactions, and hydrophobic interactions. The CDR3 of the heavy chain variable region of the antibody N1B5-h4 is involved in recognizing the HBsAg₃₄₈₋₃₅₇ peptide in MIP-H1-01 (amino acid residues 1 to 10 of MIP-H1-01) (FIG. 13). Among these, N1B5-h4 can recognize P4, T5, V6, W7, L8, S9, and V10 of HBsAg₃₄₈₋₃₅₇ (GLSPTVWLSV) (Table 11).

**Table 11. Amino acids involved in N1B5-h4/HBsAg₃₄₈₋₃₅₇ interaction and their action modes**

| MIP-H1-01 | | N1B5-h4 | | Interaction |
|---|---|---|---|---|
| Region | Residue | Region | Residue | |
| Peptide (amino acids at positions 1-10) | P4 | CDR3 | D104 | Van der Waals interaction |
| | T5 | CDR3 | G102 | Van der Waals interaction |
| | | CDR3 | P103 | Van der Waals interaction |
| | | CDR3 | D104 | Hydrogen bond |
| | | CDR3 | M105 | Hydrogen bond |
| | V6 | CDR3 | D104 | Van der Waals interaction |
| | | CDR3 | M105 | Van der Waals interaction |
| | | CDR3 | Q109 | Hydrogen bond |
| | W7 | CDR3 | G102 | Van der Waals interaction |
| | | CDR3 | M105 | Van der Waals interaction |
| | L8 | CDR3 | W100 | Hydrophobic interaction |
| | | CDR3 | L101 | Hydrophobic interaction |
| | | CDR3 | M105 | Van der Waals interaction |
| | S9 | CDR3 | D99 | Hydrogen bond |
| | | CDR3 | W100 | Van der Waals interaction |
| | | CDR3 | N118 | Hydrogen bond |
| | V10 | CDR3 | N118 | Van der Waals interaction |

The key amino acids identified in Section 3.4 were at positions 2, 4, 5, 6, 7, 8, 9, and 10 of HBsAg₃₄₈₋₃₅₇ (GLSPTVWLSV), and the key amino acids identified in Example 6 were at positions 2, 5, 6, 7, 8, 9, and 10 of HBsAg₃₄₈₋₃₅₇ (GLSPTVWLSV). The methods used in Section 3.4 and Example 6 could not distinguish whether the decrease in the ability of the antibody to bind to the alanine-scanning mutant peptides was due to the fact that the alanine-substituted peptide could not be properly loaded onto HLA (i.e., failed to bind to HLA), or whether the binding of the mutated peptide to the antibody was directly affected. The amino acid residue at position 2 of HBsAg₃₄₈₋₃₅₇ was generally the binding site for the antigen peptide to HLA (i.e., the loading site). After mutation of leucine (L) at position 2 of HBsAg₃₄₈₋₃₅₇ to alanine (A), the peptide may no longer bind to HLA. Thus, although a significant decrease in peptide-mediated activity was detected in Section 3.4 and Example 6, leucine at position 2 may not be directly involved in binding to the antibody.

The crystal structure in Example 10 showed that N1B5-h4 binds to the proline residue at position 4 (4P) of HBsAg₃₄₈₋₃₅₇, and the activity mediated by the mutation of 4P to 4A in Example 6 was not significantly reduced, indicating that the binding of N1B5-h4 to position 4 of HBsAg₃₄₈₋₃₅₇ in the complex is not stringent, and that amino acid substitutions with similar properties have a weaker effect on their interaction, particularly for molecules with a bispecific antibody structure.

All patents, patent application publications, and non-patent literature mentioned and/or listed in this application are incorporated herein by reference in their entirety. The exemplary embodiments of the inventions in the present application have been described hereinabove. However, those skilled in the art may modify or amend the exemplary embodiments described herein without departing from the spirit and scope of the present application, and variations or equivalents derived therefrom shall also fall within the scope of the present application.

### Sequence Listing

**SEQ ID NO: 32**
   TNCMG
**SEQ ID NO: 33**
   AIYTGGGRAVYADSVRG
**SEQ ID NO: 34**
   DWLGPDMTDIQVLGALPWFNY
**SEQ ID NO: 35** 
QVQLVESGGGSVQAGGSLRLSCAASDDTYMTNCMGWFRQAPGKEREGVAAIYTGGGRAVYADSVR
GRFTISQDNAKNTVYLQMNSLKPEDTAMYYCAADWLGPDMTDIQVLGALPWFNYWGQGTLVTVSS
**SEQ ID NO: 36** 
EVQLVESGGGLVQPGGSLRLSCAASDDTYMTNCMGWFRQAPGKERVGVAAIYTGGGRAVYADSVRG
RFTISQDNAKNTVYLQMNSLKPEDTAMYYCAADWLGPDMTDIQVLGALPWFNYWGQGTLVTVSS
**SEQ ID NO: 37**
   GYTMN
**SEQ ID NO: 38**
   LINPYKGVSTYNQKFKD
**SEQ ID NO: 39**
   SGYYGDSDWYFDV
**SEQ ID NO: 40**
   RASQDIRNYLN
**SEQ ID NO: 41**
   YTSRLES
**SEQ ID NO: 42**
   QQGNTLPWT
**SEQ ID NO: 43**
**SEQ ID NO: 44**
**SEQ ID NO: 45** 
QVQLVESGGGSVQAGGSLRLSCAASDDTYMTNCMGWFRQAPGKEREGVAAIYTGGGRAVYADSVR
GRFTISQDNAKNTVYLQMNSLKPEDTAMYYCAADWLGPDMTDIQVLGALPWFNYWGQGTLVTVSS
GGGGSGGGGSGGGGSQVQLVESGGGSVQAGGSLRLSCAASDDTYMTNCMGWFRQAPGKEREGVAA
IYTGGGRAVYADSVRGRFTISQDNAKNTVYLQMNSLKPEDTAMYYCAADWLGPDMTDIQVLGALPW
FNYWGQGTLVTVSSASERKSSVECPPCPAPEFEGGPSVFLFPPKPKDTLMISRTPEVTCVVVDVSQEDP
 
EVQFNWYVDGVEVHNAKTKPREEQYNSTYRVVSVLTVLHQDWLNGKEYKCKVSNKALPASIEKTIS
KAKGQPREPQVCTLPPSQEEMTKNQVSLSCAVKGFYPSDIAVEWESNGQPENNYKTTPPVLDSDGSFF
LVSKLTVDKSRWQEGNVFSCSVMHEALHNHYTQKSLSLSPGK
**SEQ ID NO: 46** 
EVQLVESGGGLVQPGGSLRLSCAASDDTYMTNCMGWFRQAPGKERVGVAAIYTGGGRAVYADSVRG
RFTISQDNAKNTVYLQMNSLKPEDTAMYYCAADWLGPDMTDIQVLGALPWFNYWGQGTLVTVSSG
GGGSGGGGSGGGGSEVQLVESGGGLVQPGGSLRLSCAASDDTYMTNCMGWFRQAPGKERVGVAAIY
TGGGRAVYADSVRGRFTISQDNAKNTVYLQMNSLKPEDTAMYYCAADWLGPDMTDIQVLGALPWF
NYWGQGTLVTVSSASERKSSVECPPCPAPEFEGGPSVFLFPPKPKDTLMISRTPEVTCVVVDVSQEDPE
VQFNWYVDGVEVHNAKTKPREEQYNSTYRVVSVLTVLHQDWLNGKEYKCKVSNKALPASIEKTISK
AKGQPREPQVCTLPPSQEEMTKNQVSLSCAVKGFYPSDIAVEWESNGQPENNYKTTPPVLDSDGSFFL
VSKLTVDKSRWQEGNVFSCSVMHEALHNHYTQKSLSLSPGK
**SEQ ID NO: 47** 
DIQMTQSPSSLSASVGDRVTITCRASQDIRNYLNWYQQKPGKAPKLLIYYTSRLESGVPSRFSGSGSGT
DYTLTISSLQPEDFATYYCQQGNTLPWTFGQGTKVEIKGGGGSGGGGSGGGGSGGGGSGGGSEVQLV
ESGGGLVQPGGSLRLSCAASGYSFTGYTMNWVRQAPGKGLEWVALINPYKGVSTYNQKFKDRFTISV
DKSKNTAYLQMNSLRAEDTAVYYCARSGYYGDSDWYFDVWGQGTLVTVSSASERKSSVECPPCPAP
EFEGGPSVFLFPPKPKDTLMISRTPEVTCVVVDVSHEDPEVKFNWYVDGVEVHNAKTKPREEQYNST
YRVVSVLTVLHQDWLNGKEYKCKVSNKALPASIEKTISKAKGQPREPQVYTLPPCREEMTKNQVSLW
CLVKGFYPSDIAVEWESNGQPENNYKTTPPVLDSDGSFFLYSKLTVDKSRWQQGNVFSCSVMHEALH
NHYTQKSLSLSPGK
**SEQ ID NO: 48** 
QDGNEEMGGITQTPYKVSISGTTVILTCPQYPGSEILWQHNDKNIGGDEDDKNIGSDEDHLSLKEFSEL
EQSGYYVCYPRGSKPEDANFYLYLRARVCENCMEMD
**SEQ ID NO: 49**
**SEQ ID NO: 50** 
GLSPTVWLSVGCGGSGGGGSGGGGSIQRTPKIQVYSRHPAENGKSNFLNCYVSGFHPSDIEVDLLKNG
ERIEKVEHSDLSFSKDWSFYLLYYTEFTPTEKDEYACRVNHVTLSQPKIVKWDRDMGGGGSGGGGSG
GGGSGGGGSGSHSMRYFFTSVSRPGRGEPRFIAVGYVDDTQFVRFDSDAASQRMEPRAPWIEQEGPE
YWDGETRKVKAHSQTHRVDLGTLRGCYNQSEAGSHTVQRMYGCDVGSDWRFLRGYHQYAYDGKD
YIALKEDLRSWTAADMAAQTTKHKWEAAHVAEQLRAYLEGTCVEWLRRYLENGKETLQRTDAPKT
HMTHHAVSDHEATLRCWALSFYPAEITLTWQRDGEDQTQDTELVETRPAGDGTFQKWAAVVVPSGQE
QRYTCHVQHEGLPKPLTLRWE
**SEQ ID NO: 51** 
MGTNLSVPNPLGFFPDHQLDPAFGANSNNPDWDFNPNKDQWPEANQVGAGAFGPGFTPPHGGLLGW
SPQAQGILTTVPAAPPPASTNRQSGRQPTPISPPLRDSHPQAMQWNSTTFHQALLDPRVRGLYFPAGGSS
SGTVNPVPTIVSPISSIFSRTGDPAPNMESTTSGFLGPLLVLQAGFFLLTRILTIPQSLDSWWTSLNFLGEA
PTCPGQNSQSPTSNHSPTSCPPICPGYRWMCLRRFIIFLFILLLCLIFLLVLLDYQGMLPVCPLLPGTSTTS
 
TGPCKTCTIPAQGTSMFPSCCCTKPSDGNCTCIPIPSSWAFARFLWEWASVRFSWLSLLVPFVQWFAGL
SPTVWLSVIWMMWYWGPSLYNILSPFLPLLPIFFCLWVYI
**SEQ ID NO: 52**
   GGGGSGGGGSGGGGS
**SEQ ID NO: 53**
   TCCTTCCCCGTCAGCCAGTCCT
**SEQ ID NO: 54**
   HHHHHH
**SEQ ID NO: 55** 
ASPRGPTIKPCPPCKCPAPNLLGGPSVFIFPPKIKDVLMISLSPIVTCVVVDVSEDDPDVQISWFVNNVE
VHTAQTQTHREDYNSTLRVVSALPIQHQDWMSGKEFKCKVNNKDLPAPIERTISKPKGSVRAPQVYV
LPPPEEEMTKKQVTLTCMVTDFMPEDIYVEWTNNGKTELNYKNTEPVLDSDGSYFMYSKLRVEKKN
WVERNSYSCSVVHEGLHNHHTTKSFSRTPGK
**SEQ ID NO: 56** 
ASTKGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKPKDTLMISRT
PEVTCVVVDVSHEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPGK
**SEQ ID NO: 57** 
ASTKGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKPKDTLMISRT
PEVTCVVVDVSHEDPEVKFNWYVDGVEVHNAKTKPREEQYNSTYRVVSVLTVLHQDWLNGKEYKC
KVSNKALPAPIEKTISKAKGQPREPQVCTLPPSREEMTKNQVSLSCAVKGFYPSDIAVEWESNGQPENN
YKTTPPVLDSDGSFFLVSKLTVDKSRWQQGNVFSCSVMHEALHNHYTQKSLSLSPGK
**SEQ ID NO: 58** 
ASTKGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKPKDTLMISRT
PEVTCVVVDVSHEDPEVKFNWYVDGVEVHNAKTKPREEQYNSTYRVVSVLTVLHQDWLNGKEYKC
KVSNKALPAPIEKTISKAKGQPREPQVYTLPPCREEMTKNQVSLWCLVKGFYPSDIAVEWESNGQPEN
NYKTTPPVLDSDGSFFLYSKLTVDKSRWQQGNVFSCSVMHEALHNHYTQKSLSLSPGK
**SEQ ID NO: 59** 
ASTKGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPEFEGGPSVFLFPPKPKDTLMISRT
PEVTCVVVDVSHEDPEVKFNWYVDGVEVHNAKTKPREEQYNSTYRVVSVLTVLHQDWLNGKEYKC
KVSNKALPASIEKTISKAKGQPREPQVCTLPPSREEMTKNQVSLSCAVKGFYPSDIAVEWESNGQPENN
YKTTPPVLDSDGSFFLVSKLTVDKSRWQQGNVFSCSVMHEALHNHYTQKSLSLSPGK
**SEQ ID NO: 60** 
ASTKGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPEFEGGPSVFLFPPKPKDTLMISRT
 
PEVTCVVVDVSHEDPEVKFNWYVDGVEVHNAKTKPREEQYNSTYRVVSVLTVLHQDWLNGKEYKC
KVSNKALPASIEKTISKAKGQPREPQVYTLPPCREEMTKNQVSLWCLVKGFYPSDIAVEWESNGQPEN
NYKTTPPVLDSDGSFFLYSKLTVDKSRWQQGNVFSCSVMHEALHNHYTQKSLSLSPGK
**SEQ ID NO: 61** 
ASTKGPSVFPLAPSSKSTSEGTAALGCLVKDYFPEPVTVSWNSGALTSGVHTFPAVLQSSGLYSLSSVVT
VPSSSLGTQTYICNVDHKPSNTKVDKTVERKSCVECPPCPAPEFEGGPSVFLFPPKPKDTLMISRTPEVT
CVVVDVSQEDPEVQFNWYVDGVEVHNAKTKPREEQYNSTYRVVSVLTVLHQDWLNGKEYKCKVSN
KALPASIEKTISKAKGQPREPQVCTLPPSQEEMTKNQVSLSCAVKGFYPSDIAVEWESNGQPENNYKTT
PPVLDSDGSFFLVSKLTVDKSRWQEGNVFSCSVMHEALHNHYTQKSLSLSPGK
**SEQ ID NO: 62** 
ASTKGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKRVERKSCVECPPCPAPEFEGGPSVFLFPPKPKDTLMISRTPEV
TCVVVDVSHEDPEVKFNWYVDGVEVHNAKTKPREEQYNSTYRVVSVLTVLHQDWLNGKEYKCKVS
NKALPASIEKTISKAKGQPREPQVYTLPPCREEMTKNQVSLWCLVKGFYPSDIAVEWESNGQPENNYK
TTPPVLDSDGSFFLYSKLTVDKSRWQQGNVFSCSVMHEALHNHYTQKSLSLSPGK
**SEQ ID NO: 63** 
GQPKAAPSVTLFPPSSEELQANKATLVCLISDFYPGAVTVAWKADSSPVKAGVETTTPSKQSNNKYAAS
SYLSLTPEQWKSHRSYSCQVTHEGSTVEKTVAPTECS
**SEQ ID NO: 64**
**SEQ ID NO: 65** 
EVQLLESGGGLVQPGGSLRLSCAASGFTFSSYAMSWVRQAPGKGLEWVSAISGSGGSTYYADSVKGR
FTISRDNSKNTLYLQMNSLRAEDTAVYYCAKGSGFDYWGQGTLVTVSS
**SEQ ID NO: 66** 
EIVLTQSPGTLSLSPGERATLSCRASQSVSSSYLAWYQQKPGQAPRLLIYGASSRATGIPDRFSGSGSGT
DFTLTISRLEPEDFAVYYCQQYGSSPLTFGQGTKVEIK
**SEQ ID NO: 67** 
AKEVEQNSGPLSVPEGAIASLNCTYSDRGSQSFFWYRQYSGKGPELIMSIYSDGDKEDGRFTAQLNKA
SQYVSLLIRDSQPSDSATYLCAARNYKTDLLIFGTGTRLQVFP
**SEQ ID NO: 68** 
NAGVTQTPKFQVLKTGQSMTLQCAQDMSHGYMSWYRQDPGMGLRLIHYSVGAGITDQGEVPNGYN
VSRSTTEDFPLRLLSAAPSQTSVYFCASSYATGGTGDLFFGEGSRLTVL
**SEQ ID NO: 69** 
AKEVEQNSGPLSVPEGAIASLNCTYSDRGSQSFFWYRQYSGKGPELIMSIYSDGDKEDGRFTAQLNKA
SQYVSLLIRDSQPSDSATYLCAARNYKTDLLIFGTGTRLQVFPYIQNPDPAVYQLRDSKSSDKFVCLFT
DFDSQINVSQSKDSDVYITDKCVLDMRSMDFKSNSAVAWSNKSDFTCANAFNNSIIPEDTFFPSPESSC
GGGGSASERKSSVECPPCPAPEFEGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPASIEKTISKAKGQPREP
QVYTLPPCREEMTKNQVSLWCLVKGFYPSDIAVEWESNGQPENNYKTTPPVLDSDGSFFLYSKLTVDK
 SRWQQGNVFSCSVMHEALHNHYTQKSLSLSPGK
**SEQ ID NO: 70** 
NAGVTQTPKFQVLKTGQSMTLQCAQDMSHGYMSWYRQDPGMGLRLIHYSVGAGITDQGEVPNGYN
VSRSTTEDFPLRLLSAAPSQTSVYFCASSYATGGTGDLFFGEGSRLTVLEDLKNVFPPEVAVFEPSKAEIS
RTQKATLVCLATGFYPPHVELSWWVNGKEVHDGVCTDPQPLKEQPALNDSRYALSSRLRVSATFWQD
PRNHFRCQVQFYGLSENDEWTQDRAKPVTQIVSAEAWGRADCGGGGSASERKSCVECPPCPAPEFEG
GPSVFLFPPKPKDTLMISRTPEVTCVVVDVSQEDPEVQFNWYVDGVEVHNAKTKPREEQYNSTYRVV
SVLTVLHQDWLNGKEYKCKVSNKALPASIEKTISKAKGQPREPQVCTLPPSQEEMTKNQVSLSCAVKG
FYPSDIAVEWESNGQPENNYKTTPPVLDSDGSFFLVSKLTVDKSRWQEGNVFSCSVMHEALHNHYTQ
KSLSLSPGK
**SEQ ID NO: 71** 
QVQLVESGGGSVQAGGSLRLSCAASDDTYMTNCMGWFRQAPGKEREGVAAIYTGGGRAVYADSVR
GRFTISQDNAKNTVYLQMNSLKPEDTAMYYCAADWLGPDMTDIQVLGALPWFNYWGQGTLVTVSS
GGGGSGGGGSGGGGSQVQLVESGGGSVQAGGSLRLSCAASDDTYMTNCMGWFRQAPGKEREGVAA
IYTGGGRAVYADSVRGRFTISQDNAKNTVYLQMNSLKPEDTAMYYCAADWLGPDMTDIQVLGALPW
FNYWGQGTLVTVSS
**SEQ ID NO: 72** 
EVQLVESGGGLVQPGGSLRLSCAASDDTYMTNCMGWFRQAPGKERVGVAAIYTGGGRAVYADSVRG
RFTISQDNAKNTVYLQMNSLKPEDTAMYYCAADWLGPDMTDIQVLGALPWFNYWGQGTLVTVSSG
GGGSGGGGSGGGGSEVQLVESGGGLVQPGGSLRLSCAASDDTYMTNCMGWFRQAPGKERVGVAAIY
TGGGRAVYADSVRGRFTISQDNAKNTVYLQMNSLKPEDTAMYYCAADWLGPDMTDIQVLGALPWF
NYWGQGTLVTVSS
**SEQ ID NO: 73** 
ASERKSSVECPPCPAPEFEGGPSVFLFPPKPKDTLMISRTPEVTCVVVDVSQEDPEVQFNWYVDGVEV
HNAKTKPREEQYNSTYRVVSVLTVLHQDWLNGKEYKCKVSNKALPASIEKTISKAKGQPREPQVCTL
PPSQEEMTKNQVSLSCAVKGFYPSDIAVEWESNGQPENNYKTTPPVLDSDGSFFLVSKLTVDKSRWQE
GNVFSCSVMHEALHNHYTQKSLSLSPGK
**SEQ ID NO: 74** 
DIQMTQSPSSLSASVGDRVTITCRASQDIRNYLNWYQQKPGKAPKLLIYYTSRLESGVPSRFSGSGSGT
DYTLTISSLQPEDFATYYCQQGNTLPWTFGQGTKVEIKGGGGSGGGGSGGGGSGGGGSGGGSEVQLV
ESGGGLVQPGGSLRLSCAASGYSFTGYTMNWVRQAPGKGLEWVALINPYKGVSTYNQKFKDRFTISV
DKSKNTAYLQMNSLRAEDTAVYYCARSGYYGDSDWYFDVWGQGTLVTVSS
**SEQ ID NO: 75** 
ASERKSSVECPPCPAPEFEGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPASIEKTISKAKGQPREPQVYTL
PPCREEMTKNQVSLWCLVKGFYPSDIAVEWESNGQPENNYKTTPPVLDSDGSFFLYSKLTVDKSRWQ
QGNVFSCSVMHEALHNHYTQKSLSLSPGK
**SEQ ID NO: 76** 
DIQMTQSPSSLSASVGDRVTITCRASQDIRNYLNWYQQKPGKAPKLLIYYTSRLESGVPSRFSGSGSGT
DYTLTISSLQPEDFATYYCQQGNTLPWTFGQGTKVEIKGGGGSGGGGSGGGGSGGGGSGGGSEVQLV
 
ESGGGLVQPGGSLRLSCAASGYSFTGYTMNWVRQAPGKGLEWVALINPYKGVSTYNQKFKDRFTISV
DKSKNTAYLQMNSLRAEDTAVYYCARSGYYGDSDWYFDVWGQGTLVTVSSGGGGSNAGVTQTPKF
QVLKTGQSMTLQCAQDMSHGYMSWYRQDPGMGLRLIHYSVGAGITDQGEVPNGYNVSRSTTEDFP
LRLLSAAPSQTSVYFCASSYATGGTGDLFFGEGSRLTVLEDLKNVFPPEVAVFEPSKAEISRTQKATLVC
LATGFYPPHVELSWWVNGKEVHDGVCTDPQPLKEQPALNDSRYALSSRLRVSATFWQDPRNHFRCQV
QFYGLSENDEWTQDRAKPVTQIVSAEAWGRADCGGGGSASERKSCVECPPCPAPEFEGGPSVFLFPPK
PKDTLMISRTPEVTCVVVDVSQEDPEVQFNWYVDGVEVHNAKTKPREEQYNSTYRVVSVLTVLHQD
WLNGKEYKCKVSNKALPASIEKTISKAKGQPREPQVCTLPPSQEEMTKNQVSLSCAVKGFYPSDIAVE
WESNGQPENNYKTTPPVLDSDGSFFLVSKLTVDKSRWQEGNVFSCSVMHEALHNHYTQKSLSLSPGK
**SEQ ID NO: 77**
   ALSPTVWLSV
**SEQ ID NO: 78**
   GASPTVWLSV
**SEQ ID NO: 79**
   GLAPTVWLSV
**SEQ ID NO: 80**
   GLSATVWLSV
**SEQ ID NO: 81**
   GLSPAVWLSV
**SEQ ID NO: 82**
   GLSPVAWLSV
**SEQ ID NO: 83**
   GLSPTVALSV
**SEQ ID NO: 84**
   GLSPTVWASV
**SEQ ID NO: 85**
   GLSPTVWLAV
**SEQ ID NO: 86**
   GLSPTVWLSA
**SEQ ID NO: 87**
   FLLTRILTI
**SEQ ID NO: 88**

EVQLVESGGGLVQPGGSLRLSCAASDDTYMTNCMGWFRQAPGKERVGVAAIYTGGGRAVYADSVRG
RFTISQDNAKNTVYLQMNSLKPEDTAMYYCAADWLGPDMTDIQVLGALPWFNYWGQGTLVTVSSS
AWSHPQFEKGGGSGGGSGGSAWSHPQFEK

### Reference

1. Leoni, M, C., Ustianowski, A., Farooq, H, et al., (2018) HIV, HCV and HBV: a review of parallels and differences. Infectious Diseases and Therapy 7(4): 407-419.
2. Lee, H, M., Banini, B, A., (2019) Updates on chronic HBV: current challenges and future goals. Curr Treat Options Gastroenterol 17:271-291.
3. Iannacone, M., Guidotti, L, G., (2022) Immunobiology and pathogenesis of hepatitis B virus infection. Nature reviews. Immunology22(1):19-32.
4. Mcnaughton, A, L., V D'Arienzo, Ansari, M, A., et al. (2018) Insights From Deep Sequencing of the HBV Genome-Unique, Tiny, and Misunderstood. Gastroenterology 156(2):384-399.
5. Stuyver, L., De, G, S., Van, G, C., et al., (2000) A new genotype of hepatitis B virus: complete genome and phylogenetic relatedness. Journal of General Virology 81(1):67-74.
6. Heléne., Norder., Berit., et al., (1993) Genetic relatedness of hepatitis B viral strains of diverse geographical origin and natural variations in the primary structure of the surface antigen. Journal of General Virology74:1341-1348.
7. Lindh., A, S., Andersson., et al., (1997) Genotypes, nt 1858 variants, and geographic origin of hepatitis B virus--large-scale analysis using a new genotyping method. The Journal of infectious diseases175: 1285-1293.
8. Chinese Society of Hepatology, Chinese Medical Associationand, Chinese Society of Infectious Diseases, Chinese Medical Association, (2022), Guidelines for the prevention and treatment of chronic hepatitis B, Chinese Journal of Hepatology, December, Volume 30, Issue 12.
9. Global progress report on HIV, viral hepatitis and sexually transmitted infections, 2021. Accountability for the global health sector strategies 2016-2021: actions for impact [EB/OL]. https://apps. who.int/iris/bitstream/handle/10665/342808/ 9789240030985-eng.pdf.
10. Philips, C, A., Ahamed, R., Abduljaleel, J, K., et al., (2021) Critical Updates on Chronic Hepatitis B Virus Infection in 2021.Cureus 13(10):19152.
11. Yixiang, X., To'A, S, G., Ningyan, Z., et al., (2019) T-cell receptor mimic (TCRm) antibody therapeutics against intracellular proteins. Antibody Therapeutics 2(1):22-32.
12. Horig, H., Lee, C, S., Kaufman, H, L. (2002) Prostate-specific antigen vaccines for prostate cancer. Expert Opin Biol Ther2(4):395-408.
13. Vlad, A, M., Kettel, J, C., Alajez, N, M., et al., (2004) MUC1 immunobiology: from discovery to clinical applications. Advances in Immunology 82:249-293.
14. Blum, J, S., Wearsch, P, A., Cresswell, P. (2013) Pathways of antigen processing. Annu Rev Immunol 31: 443-473.
15. Nayersina, R., Fowler, P., Guilhot, S., et al., (1993) HLA A2 restricted cytotoxic T lymphocyte responses to multiple hepatitis B surface antigen epitopes during hepatitis B virus infection. Journal of Immunology 150(10):4659-4671.
16. A. Margaret Merchant et al., An efficient route to human bispecific IgG, Nature Biotechnology, Volume 16, 1998.
17. Kabat, Sequences of Proteins of Immunological Interest, National Institutes of Health, Bethesda, Md. (1991).
18. A1-Lazikani et al., J. Mol. Biol. 273:927-948 (1997).
19. Martin et al., Proc. Natl. Acad. Sci.USA86:9268-9272 (1989).
20. Kotsiou, E., Brzostek, J., Lenart, I., et al., (2010) Dimerization of soluble disulfide trap single chain major histocompatibility complex class I molecules dependent on peptide binding affinity. Antioxid Redox Signal 15(3):635-644.
21. Romão, E., Poignavent, V., Vincke, C., et al. (2018) Ritzenthaler C, Muyldermans S, Monsion B. Construction of High-Quality Camel Immune Antibody Libraries. Methods Mol Biol 1701:169-187.
22. CN 201510097117.0
23. Phage Display: A Practical Approach, Clackson, T. (USA) and Lowman, H. B. (USA) (ed.), translated by Lan Ma et al., Chemical Industry Press, May 2008.5.
24. US 20160200833A1
25. WO2020193745 A1
26. Tan, P., Mitchell, D, A., Buss, T, N., et al. (2022) "Superhumanized" antibodies: reduction of immunogenic potential by complementarity-determining region grafting with human germline sequences: application to an anti-CD28. J Immunol 169(2):1119-1125.
27. US 5821337A

## Claims

1. A bispecific antibody comprising a first antigen-binding fragment that binds to an HLA-A02/HBsAg complex and a second antigen-binding fragment that binds to an activated T-cell antigen;
preferably, the first antigen-binding fragment binds to the HLA-A02/HBsAg complex at an epitope comprising one or more residues at positions 348-357 of HBsAg as set forth in SEQ ID NO: 51;
more preferably, the first antigen-binding fragment binds to the HLA-A02/HBsAg complex at an epitope comprising at least one of residues at positions 349, 351, 352, 353, 354, 355, 356, and 357 of HBsAg as set forth in SEQ ID NO: 51; and/or
preferably, the activated T-cell antigen is a CD3 molecule.

2. The bispecific antibody of claim 1, wherein
the first antigen-binding fragment comprises HCDR1 as set forth in SEQ ID NO: 32, HCDR2 as set forth in SEQ ID NO: 33, and HCDR3 as set forth in SEQ ID NO: 34;
preferably, the first antigen-binding fragment is in the form of a single-domain antibody;
more preferably, the first antigen-binding fragment comprises a monovalent or multivalent single-domain antibody that binds to HLA-A02/HBsAg complex; and/or
the second antigen-binding fragment comprises HCDR1 as set forth in SEQ ID NO: 37, HCDR2 as set forth in SEQ ID NO: 38, HCDR3 as set forth in SEQ ID NO: 39, LCDR1 as set forth in SEQ ID NO: 40, LCDR2 as set forth in SEQ ID NO: 41, and LCDR3 as set forth in SEQ ID NO: 42;
preferably, the second antigen-binding fragment is in the form of a single-chain variable fragment (scFv) or a Fab fragment;
more preferably, the second antigen-binding fragment is in the form of an scFv;
wherein the amino acid sequences of the HCDRs are defined according to the Kabat numbering scheme.

3. The bispecific antibody of claim 1 or 2, wherein the first antigen-binding fragment comprises a divalent single-domain antibody that binds to HLA-A02/HBsAg complex;
optionally, the first antigen-binding fragment comprises two heavy chain variable regions from monovalent single-domain antibodies that bind to the HLA-A02/HBsAg complex;
preferably, the first antigen-binding fragment comprises two heavy chain variable regions from monovalent single-domain antibodies that bind to the HLA-A02/HBsAg complex, which are linked via direct fusion; or two heavy chain variable regions from monovalent single-domain antibodies that bind to HLA-A02/HBsAg complex, which are linked via a linker;
more preferably, the linker comprises a GS-type flexible peptide, such as (GGGGS)n, where n is an integer ≥ 1.

4. The bispecific antibody of any one of claims 1 to 3, wherein
the first antigen-binding fragment comprises the amino acid sequence as set forth in SEQ ID NOs: 35, 36, 71, or 72; and/or
the second antigen-binding fragment comprises a heavy chain variable region having the amino acid sequence as set forth in SEQ ID NO: 43 and a light chain variable region having the amino acid sequence as set forth in SEQ ID NO: 44.

5. The bispecific antibody of any one of claims 1 to 4, wherein the first antigen-binding fragment comprises the amino acid sequence as set forth in SEQ ID NO: 35; and the second antigen-binding fragment comprises a heavy chain variable region having the amino acid sequence as set forth in SEQ ID NO: 43 and a light chain variable region having the amino acid sequence as set forth in SEQ ID NO: 44; or
the first antigen-binding fragment comprises the amino acid sequence as set forth in SEQ ID NO: 36; and the second antigen-binding fragment comprises a heavy chain variable region having the amino acid sequence as set forth in SEQ ID NO: 43 and a light chain variable region having the amino acid sequence as set forth in SEQ ID NO: 44; or
the first antigen-binding fragment comprises the amino acid sequence as set forth in SEQ ID NO: 71; and the second antigen-binding fragment comprises a heavy chain variable region having the amino acid sequence as set forth in SEQ ID NO: 43 and a light chain variable region having the amino acid sequence as set forth in SEQ ID NO: 44; or
the first antigen-binding fragment comprises the amino acid sequence as set forth in SEQ ID NO: 72; and the second antigen-binding fragment comprises a heavy chain variable region having the amino acid sequence as set forth in SEQ ID NO: 43 and a light chain variable region having the amino acid sequence as set forth in SEQ ID NO: 44.

6. The bispecific antibody of any one of claims 1 to 5, wherein
the first antigen-binding fragment and the second antigen-binding fragment are linked via an Fc fragment of an antibody heavy chain constant region comprising a first Fc fragment and a second Fc fragment;
preferably, the Fc fragment of the antibody heavy chain constant region is an Fc fragment of IgG1 subtype;
more preferably, the Fc fragment of the antibody heavy chain constant region is an Fc fragment of IgG1m3 allotype;
wherein the first Fc fragment comprises the amino acid C at position 354 and the amino acid W at position 366; or the amino acid C at position 349, the amino acid S at position 366, the amino acid A at position 368, and the amino acid V at position 407; and the second Fc fragment comprises amino acid C at position 354 and the amino acid W at position 366; or the amino acid C at positions 349, the amino acid S at position 366, the amino acid A at position 368, and the amino acid V at position 407;
preferably, the first Fc fragment comprises amino acid C at position 354 and the amino acid W at position 366, and the second Fc fragment comprises the amino acid C at position 349, the amino acid S at position 366, the amino acid A at position 368, and the amino acid V at position 407; and/or
the first Fc fragment and the second Fc fragment comprise the amino acid F at positions 234, the amino acid E at position 235, and the amino acid S at position 331, respectively; and/or
one of the first Fc fragment and the second Fc fragment is linked to the first antigen-binding fragment and the other of the first Fc fragment and the second Fc fragment is linked to the second antigen-binding fragment;
wherein the amino acid positions of the antibody constant regions are determined according to the EU numbering scheme.

7. The bispecific antibody of any one of claims 1 to 6, comprising a first arm that binds to the HLA-A02/HBsAg complex and a second arm that binds to the activated T-cell antigen, wherein
the first arm comprises the amino acid sequence as set forth in SEQ ID NOs: 45 or 46; and
the second arm comprises the amino acid sequence as set forth in SEQ ID NO: 47.

8. A nucleic acid molecule encoding the bispecific antibody of any one of claims 1 to 7.

9. A pharmaceutical composition comprising the bispecific antibody of any one of claims 1 to 7, and a pharmaceutically acceptable excipient, diluent, or carrier.

10. The pharmaceutical composition of claim 9, for use in the prevention or treatment of a disease caused by HBV infection in a patient who is HLA-A02/HBsAg-positive;
preferably, the disease caused by HBV infection is selected from the group consisting of hepatitis B, liver cirrhosis, liver fibrosis, and liver cancer.

11. Use of the bispecific antibody of any one of claims 1 to 7, or the pharmaceutical composition of claim 9 or 10 in the manufacture of a medicament for the prevention or treatment of a disease caused by HBV infection in a patient who is HLA-A02/HBsAg-positive;
preferably, the disease caused by HBV infection is selected from the group consisting of hepatitis B, liver cirrhosis, liver fibrosis, and liver cancer.

12. A method for preventing or treating a disease caused by HBV infection in a patient who is HLA-A02/HBsAg-positive, comprising administering to a subject in need thereof the bispecific antibody of any one of claims 1 to 7, or the pharmaceutical composition of claim 9 or 10;
preferably, the disease caused by HBV infection is selected from the group consisting of hepatitis B, liver cirrhosis, liver fibrosis, and liver cancer.
